# EUROPEAN PATENT SPECIFICATION

(11) **EP 3 638 772 B1**
(45) Date of publication and mention of the grant of the patent: **28.08.2024**
(21) Application number: 18733219.2
(22) Date of filing: 15.06.2018
(51) Int. Cl.: C12N 5/00, C12N 5/071, G01N 33/50

(54) **CELL CULTURE MEDIA FOR DIFFERENTIATION OF STEM CELLS INTO HEPATOCYTES**
ZELLKULTURMEDIEN ZUR DIFFERENZIERUNG VON STAMMZELLEN IN HEPATOZYTEN
MILIEUX DE CULTURE CELLULAIRE POUR LA DIFFÉRENCIATION DE CELLULES SOUCHES EN HÉPATOCYTES

(30) Priority: 16.06.2017 GB 201709617; 31.07.2017 LU 100352
(43) Date of publication of application: 22.04.2020
(73) Proprietor: Katholieke Universiteit Leuven, 3000 Leuven (BE)
(72) Inventor: BOON, Ruben, 3071 Erps-Kwerps (BE); VERFAILLIE, Cathérine, 3000 Leuven (BE)
(74) Representative: Arnold & Siedsma
(86) International application number: PCT/EP2018/066010
(87) International publication number: WO 2018/229274

(56) References cited:
- WO-A2-2006/026408
- MITAKA T ET AL: "Amino acid-rich medium (Leibovitz L-15) enhances and prolongs proliferation of primary cultured rat hepatocytes in the absence of serum", JOURNAL OF CELLULAR PHYSIOLOGY, vol. 147, no. 3, June 1991 (1991-06-01), pages 495 - 504, XP055465978, ISSN: 0021-9541, DOI: 10.1002/jcp.1041470316
- SIGMA-ALDRICH: "L-15 Medium Leibovitz with L-glutamine Product Number L4386", 2009, XP055466008, Retrieved from the Internet <URL:https://www.sigmaaldrich.com/content/dam/sigma-aldrich/docs/Sigma/Product_Information_Sheet/1/l4386pis.pdf> [retrieved on 20180410]
- GRUNNET N ET AL: "Maintenance of Alcohol Dehydrogenase Activity in Long-Term Culture of Hepatocytes from Female Rat", ALCOHOLISM: CLINICAL AND EXPERIMENTAL RESEARCH, vol. 13, no. 1, January 1989 (1989-01-01) - February 1989 (1989-02-01), pages 25 - 28, XP055500770, ISSN: 0145-6008, DOI: 10.1111/j.1530-0277.1989.tb00278.x
- JALEEL A & NAIR K S: "Identification of multiple proteins whose synthetic rates are enhanced by high amino acid levels in rat hepatocytes", AMERICAN JOURNAL OF PHYSIOLOGY: ENDOCRINOLOGY AND METABOLISM., vol. 286, no. 6, June 2004 (2004-06-01), pages E950 - E957, XP055501435, ISSN: 0193-1849, DOI: 10.1152/ajpendo.00403.2003
- ZHANG ZJ ET AL: "The role of amino acids in ApoB100 synthesis and catabolism in human HepG2 cells", JOURNAL OF BIOLOGICAL CHEMISTRY, vol. 268, no. 36, 25 December 1993 (1993-12-25), pages 26920 - 26926, XP055501452
- TOMIZAWA M ET AL: "Improved Survival and Initiation of Differentiation of Human Induced Pluripotent Stem Cells to Hepatocyte-Like Cells upon Culture in William's E Medium followed by Hepatocyte Differentiation Inducer Treatment", PLOS ONE, vol. 11, no. 4, E0153435, 13 April 2016 (2016-04-13), XP055501440, DOI: 10.1371/journal.pone.0153435
- REINHART D ET AL: "Benchmarking of commercially available CHO cell culture media for antibody production", APPLIED MICROBIOLOGY AND BIOTECHNOLOGY, vol. 99, no. 11, 7 April 2015 (2015-04-07), pages 4645 - 4657, XP035502970, ISSN: 0175-7598, DOI: 10.1007/S00253-015-6514-4
- LING C T ET AL: "Chemically characterized concentrated corodies for continuous cell culture (the 7C's culture media)", EXPERIMENTAL CELL RESEARCH, vol. 52, no. 2-3, October 1968 (1968-10-01), pages 469 - 489, XP024792176, ISSN: 0014-4827, [retrieved on 19681001], DOI: 10.1016/0014-4827(68)90489-8

## Description

### FIELD OF THE INVENTION

The present invention describes culture media for inducing /maintaining a mature hepatocyte phenotype or mitochondrial activity.

### BACKGROUND OF THE INVENTION

Despite improved preclinical *in vitro* models and animal models, very high drug attrition still occurs at various stages of drug development, dramatically escalating costs of developing new therapeutic agents Causes of drug failure are toxicity as well as poor efficacy. The main target organ involved in drug toxicity and failure is the liver, as hepatocytes metabolize many (pre)drugs to create active drug substances, and chemicals themselves or their metabolites can be toxic for hepatocytes. Because drug metabolisation enzymes differ significantly between species, animal studies cannot fully predict human drug toxicity and bioavailability. Therefore, primary human hepatocytes (PHHs) remain the gold standard cells for *in vitro* screening systems ¹. However, liver donors are in short supply, and significant differences exist between donors, resulting in the need to extensively screen available PHH batches. In addition, PHHs quickly lose most mature functions upon culture, and cannot be used for longer term studies ². Currently, *in vitro* drug toxicity assays use HepG2 hepatoma cells, even if they have minimal drug metabolisation capacity ³. For these reasons other immortal lines, such as HepaRG cells or transiently immortalized PHHs that possess some, but not all, *in vitro* drug metabolizing capacity, are being evaluated for their suitability for drug metabolisation and toxicity studies ⁴⁵.

Hepatocytes derived from pluripotent stem cells (PSC) might be a valid alternative for drug toxicity and metabolisation studies. Advantages of PSC-derived hepatocytes are that large renewable batches of cells could be generated, obviating the need for batch testing; while at the same time retaining the possibility of obtaining cells from individuals with different drug metabolisation and toxicity characteristics ⁶. Others and we have developed protocols to differentiate PSCs to cells that have many features of mature hepatocytes. These include albumin secretion and glycogen storage, susceptibility to infection with hepatotropic viruses ⁷⁸; and creation of liver disease models from patient derived induced (i)PSC) ⁸ Nevertheless, the major hurdle in using these cells in drug development remains the immature phenotype of PSC-derived hepatocytes, which have therefore been termed "hepatocyte like cells" or "HLCs". Compared with PHHs, HLCs express low levels of hepatocyte associated transcription factors (TFs), have low drug metabolizing capacities, and are epigenetically immature. (Baxter et al., 2015; ⁹).

One approach used to enhance hepatocyte differentiation from PSCs is the overexpression of "missing" hepatocyte TFs, commonly done using viral vectors ¹⁰ Although some maturation has been observed, this approach has drawbacks. Due to multiple copy insertion of the vectors the viral vector overexpression usually leads to TF expression levels that are hundred to thousand times higher than in PHHs. In addition, the number of copies per cell is variable, and depending on the integration site, silencing may occur in some but not all cells. A second approach used to enhance differentiation, is by optimizing the culture system to more closely mimic the liver sinusoid. These studies include co-culture of different liver resident cells (e.g. endothelium, mesenchymal cells and primary hepatocytes), recreating the soft liver mechano-biological characteristics (e.g. by using sandwich cultures of hydrogels), and/or recreating liver sinusoidal perfusion (e.g. by using microfluidic devices). All these approaches improve hepatocyte differentiation from PSCs, although HLC maturity remains below that observed in PHHs ¹¹¹²¹³.

One component of the culture system that has received significantly less attention is the nutrient microenvironment. It is known that medium composition can greatly affect cellular behaviour *in vitro.* Several studies demonstrated that nutrient metabolisation is one of the major regulators of stem cell fate, as for instance central carbon metabolism plays an important role in PSC maintenance, proliferation, differentiation and lineage specialization ¹⁴¹⁵¹⁶. Even though hepatocytes are among the most metabolically active cell types, which for instance is responsible for regulation of glucose homeostasis and detoxifying nitrogen, little attention has been paid to the metabolic profile of hepatocytes *in vitro.* Recreation of hepatocyte-specific nutrient utilization would improve PSC-hepatocyte maturation.

Drug-induced liver injury (DILI) is one of the most common causes of termination of drug development, a major reason for refusal of market authorization and for restricted use, and the leading cause of post-market drug withdrawals.

Many drugs are "pro-drugs", i.e. they need to be metabolised to create a functional chemical entity, which occurs chiefly in the liver via Phase I and Phase II metabolisation drugs. Animal models predict toxicity in humans only poorly, mostly because metabolisation and drug transport proteins differ significantly between man and rodents (the commonly used preclinical animal models), and differences in drug uptake and conversion leads to differences in drug availability in men and rodents. Therefore, alternative methodologies have been investigated to better predict drug toxicity, uptake and metabolisation. Since all hepatocyte models, including cell lines, pluripotent stem cell derived hepatocytes and cultured primary cells do not display Phase I and Phase II metabolisation in vitro, there are currently no in vitro test models to screen for hepatotoxins. Currently, primary human hepatocytes constitute the most accepted human model system, but these cells lose their drug metabolizing capacities quickly upon plating. As not only drug metabolizing capacities but also other hepatic features, including gluconeogenic capacity, urea cycle functionality, mitochondrial activity and expression of certain hepatic transcription factors is lost upon culture no current hepatocyte model recapitulates hepatocyte biology in vitro. Similarly, hepatocytes generated in vitro from pluripotent stem cells do not acquire the ability to perform these functions, do not express the entire panel of hepatic transcription factors, and stay metabolically immature (utilize glycolysis as their primary source of energy). Also, all available cancer- and immortalized liver cell lines do not possess mature hepatic functions, therefore their power predicting liver toxicity is limited.

### SUMMARY OF THE INVENTION

We here describe that although induced expression of TFs enhances hepatic progeny maturation, PSC-derived hepatic progeny with mature cell-metabolism properties (gluconeogenesis and mitochondrial activity) and drug metabolisation activity similar to those of freshly plated PHHs can only be obtained when TF induction is combined with nutrient optimization. We demonstrate that supplementation of culture medium with one or more amino acids is required to provide carbons and nitrogen for glucose and urea secretion, and is able to actively induce cytochrome P450 enzymes, and this in an Mechanistic target of rapamycin (mTOR) dependent manner. Moreover we found this mechanism to be also present in commonly used hepatoma cell lines, thus allowing for the optimization of a high throughput drug screening system that resembles toxicity of freshly isolated primary hepatocytes.

Hepatic cell models including primary hepatocytes, pluripotent stem cell derived hepatocyte-like cells (HLCs), and hepatoma cell lines all display an immature profile *in vitro* and possess insufficient drug metabolizing capacities to accurately predict liver toxicity. We here show that this immature functional profile is also characterized by an immature nutrient utilization characterized by high levels of glycolysis and low levels of mitochondrial activity. We found these features to be correlated with overall hepatic maturity as the overexpression of several hepatic transcription factors from the AAVS1 locus of pluripotent stem cells not only resulted in increased expression of drug metabolizing enzymes, but also in a nutrient switch towards pyruvate oxidation. Furthermore, we found that current culture conditions, even under conditions of TF overexpression, were limiting to fully induce an amino acid fuelled metabolism characterized by glucose and urea secretion and high mitochondrial activity. We also show that different concentrations of AA's have a profound effect on hepatocyte maturation. More specifically, we optimized culture conditions that favor a gluconeogenic profile, and conditions that massively induce the expression of drug metabolizing enzymes in an mTORC dependent manner. These optimized media conditions allowed us to establish a high throughput screening system that predicts hepatic toxicity at concentrations, which are similar to freshly isolated hepatocytes, using either PSC derived HLCs or commonly used hepatoma cell lines as a model. This work shows that metabolism and nutrient dependence are essential drivers of hepatocyte maturation, are essential for the prediction of cellular toxicity, and should be considered in any differentiation protocol. Furthermore, this systems will prove essential in pre-screening pharmaceutical compounds for drug induced liver injury.

The present invention discloses culture media that induce a mature metabolic profile in pluripotent stem cell derived hepatocytes. Addition of high concentrations of amino acids at the end of differentiation, i.e. after hepatoblast specification was found to induce gluconeogenesis and glucose independence, mitochondrial metabolism and urea secretion in pluripotent stem cell derived hepatocytes, thus recapitulating essential metabolic functions of hepatocytes in vitro.

The present invention discloses culture media that induces a mature drug metabolizing profile in pluripotent stem cell derived hepatocytes and hepatic cell lines. These media induce the expression and activity of drug metabolizing enzymes in PSC derived hepatocytes and hepatic cell lines. Furthermore under these conditions pluripotent stem cell derived hepatocytes and hepatic cell liens were found to display toxicity to drugs at same levels as the current gold standard, i.e. freshly plated primary hepatocytes. Last this phenotype was, unlike primary cells, found to be stable over time as activity and expression of drug metabolizing enzymes remained stable over 10 days for pluripotent stem cell derived hepatocytes and 1 week for hepatic cell lines, and toxicity was found to be similar at different days of treatment. The media of the present invention allow studying hepatocyte specific metabolism in vitro. As this medium induces gluconeogenesis to similar levels as primary cells in pluripotent stem cell derived hepatocytes, a feature not yet observed in vitro. The present invention allows studying the regulation and general biology of hepatocyte metabolism in vitro.

The media of the present invention induce mitochondrial metabolism in hepatocytes, allowing to study mitochondrial function in hepatocytes in vitro.

The media of the present invention allow to screen for liver toxicity in vitro. As this medium induces drug metabolizing activity in pluripotent stem cell derived hepatocytes and hepatoma cell lines to levels also found in freshly plated primary hepatocytes, and allows for screening for drug toxicity in all tested cells, such as tumour cell lines.

The media of the present invention induces hepatic cells to respond in vitro to the same level as freshly isolated primary hepatocytes with regards to their metabolic profile (nutrient dependency), drug metabolisation and drug toxicity. The present invention allows the use of pluripotent stem cell derived hepatocytes and hepatic cell lines to be used as in vitro models for hepatocyte biology.

The present invention discloses that optimization of the culture medium by the addition of amino acids greatly induces hepatocyte maturity to levels which are suitable for modelling hepatocyte metabolism, drug metabolisation and drug toxicity in vitro, and this in pluripotent stem cell derived hepatocytes and hepatic cell lines. optimized culture conditions favour a gluconeogenic profile, mitochondrial activity, urea secretion and conditions that massively induce the expression of drug metabolizing enzymes in an mTORC dependent manner. These optimized media conditions allow to establish a high throughput screening system that predicts hepatic toxicity using either PSC derived HLCs or commonly used hepatoma cell line at concentrations similar to those used with freshly isolated hepatocytes. Moreover, these mature features remain stable for at least 2 weeks in culture and even increase over time.

Supplementing the media with at least 3 mg/ml amino acids induces gluconeogenesis, mitochondrial activity and glucose independence in pluripotent stem cell derived hepatocytes. Further increasing the concentration of amino acids to more than 20 mg/ml induces drug metabolizing enzymes and allowed the cells to recapitulate hepatocyte toxicity in vitro, and this in both pluripotent stem cell derived hepatocytes and hepatic cell lines.

The invention makes it feasible to develop drug screening platforms for hepatic toxicity for drug development and drug toxicity testing, and therefore is commercially relevant.

### DETAILED DESCRIPTION

Short description of the figures:
Figure 1 A: expression of AAT and NTCP upon differentiation of PSC-derived HLC, compared with primary human hepatocytes (PHHs ).
Figure 1B: expression of albumin (ALB) or cytochrome P450 (CYP)3A4 upon differentiation of PSC-derived HLC, compared with primary human hepatocytes (PHHs)
Figure 1C Functional measurement of albumin secretion and metabolisation of BFC by CYP3A4 and CYP1A2 in differentiated HLCs (D20), PHHs and 12 hour plated PHHs (PHH12).
Figure 1D: decrease of hepatocyte markers during cultivation of PHH (12, 36 and 72 hours)
Figure 2A: WGCNA analysis for genes differentially expressed between HLCs to PHHs.
Figure 2B: Expression of representative genes involved energy metabolism during differentiation of PSC, compared with primary human hepatocytes (PHHs ).
Figure 2C: Glucose uptake of differentiating PSC (DIFF) and primary human hepatocytes (PHHs ).
Figure 2D: Oxygen consumption of PSC (DO) and differentiated cells (D20) and primary human hepatocytes (PHHs), after addition of oligomycin, FCCP and antimycin.
Figure 2E: Expression of glycolytic and gluconeogenic transcripts in PHH after plating (12, 36 and 72 h)
Figure 3A: Marker expression in standard cells and cells that are genetically engineered to overexpress hepatic transcription factors (HC3X) and primary human hepatocytes (fresh PHH) and after 12h cultivation (PHH12)
Figure 3B Increased function of cells overexpressing hepatic transcription factors.
Figure 3C: Reduction of glucose uptake and illustration of "glucose independence" as cell survival in the presence (CTL) and absence of glucose (-GLUC) for HLC and HC3X cells.
Figure 3D: Oxygen consumption of differentiated cells (D20) and HC3X progeny cultured with (HC3X) or without (HC3X-GLC) glucose, and primary human hepatocytes (PHHs), after addition of oligomycin, FCCP and antimycin.
Figure 3E: illustrates 13C labelling of intracellular metabolites derived from nutrients.
Figure 3F:Shows concentrations of glucose, pyruvate and various amino acids present in the culture medium after 24 hours of culture.
Figure 4A: shows marker expression for gluconeogenic genes after induction in different media: Hepatocyte Maintenance Medium based on William's E formulation (782 mg/l Amino acids)(WE), liver differentiation medium (LDM) (214 mg/l amino acids), supplemented with increasing concentrations of Amino acids ( AA1=649.62mg/l, AA2= 1955.62mg/l and in AA3 =3696.97 mg/l).
Figure 4B shows glucose uptake in cell treated with 24 mg amino acids/ ml medium.
Figure 4C Oxygen consumption of differentiated cells (D20) and HC3X progeny cultured in the presence of AA3 without glucose (AA3 -GLC), and primary human hepatocytes (PHHs), after addition of oligomycin, FCCP and antimycin.
Figure 4D: 13C labelling of intracellular metabolites in cell types treated with AA3 medium
Figure 4E shows urea secretion (4D) of cells cultured in AA3-supplemented medium
Figure 5A: Increased expression of CYP3A4 in HLCs (D20) and HC3X progeny cultured in medium supplemented with AAG3 and 20g/l of glycine (AAGLY)
Figure 5B: Induction of CYP3A4 when replacing glycine with any of the mentioned amino acids.
Figure 5C: Induction of other CYP450 isoforms after culture in medium supplemented wit hAA3 and 20g/l of glycine (AAGLY).
Figure 5D: Relative metabolisation of BFC in Cells grown in control medium or medium supplemented with AAGLY and compared to PHHs.
Figure 5E: Western blot analysis showing mTORC activation only at the highest AA concentrations (AAGLY).
Figure 5F: Mitochondrial abundance as measured by intensity of TOMM20 staining in cells grown in control medium and in medium supplemented with AAGLY.
Figure 6A Analysis on de-differentiating hepatocytes showing the deregulated transcription factors with the highest amount of target genes.
Figure 6B and 6C. gene expression analysis showing down-regulation of the PGC1a pathway upon culture of PHHs.
Figure 6D and 6E: Time dependent maturation of HC6X cells grown in AAGly supplemented medium as measured by CYP3A4 gene expression and function
Figure 7: Toxicity curves for HC6X cells generated in two different PSc backgrounds, and grown in AAGLY supplemented medium after exposure to the hepatotoxins APAP, amiodarone and rotenone. The comparison of LD50 values is made with PHHs.
Figure 8A and 8C: Expression of CYP450 variants after exposure of the hepatoma cell lines HepG2 and HUH7, and the embryonic kidney cell line HEK293, to medium supplemented with AAGLY for 7 days.
Figure 8B: Induction of CYP3A4 when replacing glycine with any of the mentioned amino acids.
Figure 8D: Time dependent maturation of HepG2 cells grown in AAGly supplemented medium as measured by CYP3A4 gene expression
Figure 8E: Mitochondrial abundance as measured by intensity of TOMM20 staining in cells grown in control medium and in medium supplemented with AAGLY.
Figure 9: Toxicity curves for hepG2 cells
Figure 10A and B: Relative expression of PGC-1A related genes (A) and mature cardiomyocyte genes (B) in PSC-derived cardiomyocytes grown in AAGLY supplemented medium

The present invention relates to methods of differentiating stem cells into hepatocytes with one or markers of a mature hepatocyte phenotype.

Herein amino acids are present in concentration well above any concentration of amino acids that has been used in media for differentiating stem cells. The total concentration of amino acids can range from about 10, 12, 15 mg/ml medium to up to about 20, 25, 30 or even higher concentrations. Ranges of one of the above lower limits and upper limits are envisaged.

In the present invention "about" refers to a deviation of 10 % of a certain value. E.g. "bout 20" means between "18 and 22 °

Mature hepatocytes phenotype refers to properties such as expression of drug metabolizing enzymes, mitochondrial activity and overall response to hepatotoxins as encounter in primary hepatocytes.

Typical markers for determining this phenotype are albumin secretion, CYP450 activity, glycogen storage, susceptibility to infection with hepatotropic viruses, drug metabolising capacities and expression of hepatic markers such as NTCP, HNF4A and AAT.

Parameters to measure mitochondrial activity or abundance are for example TOMM20 expression, oxygen consumption rates, expression of transcription factor A, mitochondrial (TFAM), mitochondrial proteins COX1, CYTC and ATP5A1, or CYP450 (or isoforms) activity.

Typically the medium will comprise a basic mixture of all amino acids at a concentration of about 1 mg/ml medium up to 5 mg/ml. In this mixture the concentration of the amino acid at the lowest concentration may be up to 10 or 15 times lower than the concentration of the amino acid at the highest concentration (see for example table 1, wherein the concentration of Trp is about 12 times lower than the concentration of Asp.

In addition to the mixture there is typically an excess of 1, 2, 3, 4 or even 5 amino acids at a concentration which is 1, 2, 4, 5, 6, 7, 8, 9 up to 10 times higher than the concentration of the mixture of amino acids. As an example table 1 shows a mixture amino acids of 4 mg/ml and a 5 fold excess of 20 mg/ml glycine, resulting in a total of 24 mg/ml.

The excess can be obtained by the addition of one amino acid or by more amino acids, and this in equal amounts or in varying amounts (e.g. 5 mg/ml Gly or 2 mg/ml Gly and 3 mg/ml Ala or 1 mg/ml Gly and 4 mg/ml Ala).

Alternatively expressed, at least 50 %, at least 60%, or at least 70 % (w/w) of the total concentration of amino acids in the medium is represented by one up to five amino acids.

Typically the amino acids in excess are selected from the group consisting of Gly, Ala, Ser Val, Ile, Leu, Pro, Asp, taurine, Glu, Asn, and Gin, and more typically of the group consisting of Gly, Ala, Ser Val, Ile, Leu, Pro, Asp and taurine.

Amino acids which may a be present in the basic mixture of all amino acids but which are not added as excess are typically Thr, Tyr, Cys, Met, Arg, His, Phe, Lys or Trp.

As indicated above, apart from the 20 amino acids which are incorporated in proteins also taurine can be used in the media and methods of the present invention. Accordingly it is envisaged that other amino acids such as phosphoserine, phosphoethanolamine, alpha-amino adipic acid, citrulline, α-aminobutyric acid, Norleucine, beta-alanine, 1-methyl histidine or 3-methyl histidine or other physiological amino acids can be used.

Without being bound by theory, it is assumed that insufficient nutrients impede the differentiation of cells into a hepatocyte with a mature phenotype. The methods and compositions are therefore suitable in the cultivation and differentiation of any type of progenitor or stem cells into hepatocytes with a mature phenotype. The methods and compositions of the present invention are particularly suitable in the differentiation of stem cells such as PSC and iPSC. Generally these cells are human cells, but any other mammalian cell can be equally used.

The process of differentiation of human PSC into hepatocytes takes about 20 days. The need of nutrients to sustain the metabolism of hepatocytes is especially relevant when the cells become more differentiated. Thus although the medium with high amino acid content is generally used throughout the entire cultivation from stem cell to hepatocyte, the medium can also be applied during a part of the differentiation process, and this generally during the last part of the differentiation, for example during the last 8 days of differentiation or during the last 4 days of differentiation. Furthermore, differentiated cells, i.e. cells with a marker of a mature hepatocyte phenotype, can be further maintained in a high amino acid medium to prevent de-differentiation.

It has been found that the beneficial effect of high concentrations of amino acids is obtained in a variety of cells using different media. Culture media for specific cell types have been described in the art and are known to the skilled person, and merely request the addition of amino acids. Accordingly, a culture medium typically refers to serum free or serum comprising media known in the art for a certain cell type. This medium can be based on a variety of available formulations such as Basal Medium Eagle, William's medium E formulation LeibovitZ L-15 media, can contain cytokines such as hepatocyte growth factor and/or epidermal growth factor, can contain glucose or be glucose free, can contain fatty acids and can contain vitamins and other supplements such as insulin, transferrin, dexamethasone and others.

Further disclosed herein is a method to determine the time needed for differentiation of stem cells into hepatocytes with a marker of a mature phenotype, by differentiating cells in a high amino acid medium and determining the appearance of a marker of a mature hepatocyte. The supply of the required high nutrients facilitates the metabolism such that the cells in the presence of high concentrations of amino acids reach the stage of cells expressing a marker of a mature hepatocyte cell at an earlier time point than prior art methods. Depending on the specific amino acids composition the time period for differentiation may be shortened from 20 down to 19, 18, 17, 16, 15 or even 14 days.

Various markers for a mature phenotype of a hepatocyte are known in the art and described in the examples section of the present invention. Typical markers are albumin secretion, urea secretion, CYP450 (e.g.CYP3A4) activity, glycogen storage, susceptibility to infection with hepatotropic viruses, drug metabolising capacities, expression of NTCP, HNF4A, AAT and other markers which are described in the examples section

As explained in the examples the effect of the high amino acid concentration can be even improved when stem cells are transfected with certain transcription factors. Apart from 3 transcription factors known in the art, the present invention foreseen up to 3 additional transcriptions factors as indicated in the examples.

Also disclosed herein is a culture media characterised in the presence of at least 3 mg amino acids/ml medium. The concentration and composition of the amino acids has been described in detail in the above section

Another aspect relates to the use of the above media for the differentiation of stem cells into hepatocyte cells with one or markers of a mature hepatocyte phenotype.

Another aspect of the present invention relates to the use of the above media for the for maintaining the phenotype of cells expressing one or more markers of a mature hepatocyte phenotypes. Such cells include partially or fully differentiated stem cells, progenitors cells or precursor cells, freshly isolated and cultivated hepatocyte cells as well as primary tumour cells or immortalised tumour cell lines.

The present invention shows embodiments of media which allow successful differentiation of stem cells into hepatocytes. Alternative embodiments of media can be determined and formulated by contacting differentiating stem cells with a test medium and essaying for marker of a mature hepatocyte phenotype.

Another aspect of the invention thus relates to methods for the identification of a medium suitable for the differentiation of stem cells into hepatocytes with expressing one or more markers of a mature hepatocyte phenotype comprising the steps of:
- providing a test medium with one or more amino acids at determined concentrations wherein the total concentration of amino acids in said medium is at least 3, 5, 10, 15 or 20 mg/ml,
- differentiating the stems cells into hepatocytes in the presence of said test medium,
- determining one or more markers for a mature phenotype of hepatocytes,
- selecting a medium, wherein the presence of such one or more parameters is determined, as suitable for the differentiation stem cells into hepatocytes with a mature phenotype.

### EXAMPLES

### Reference

### Example 1: Media improves function of PSC-derived hepatocytes

### Differentiation of PSC to the hepatic lineage using growth factors yields progeny that is functional as well as metabolic immature.

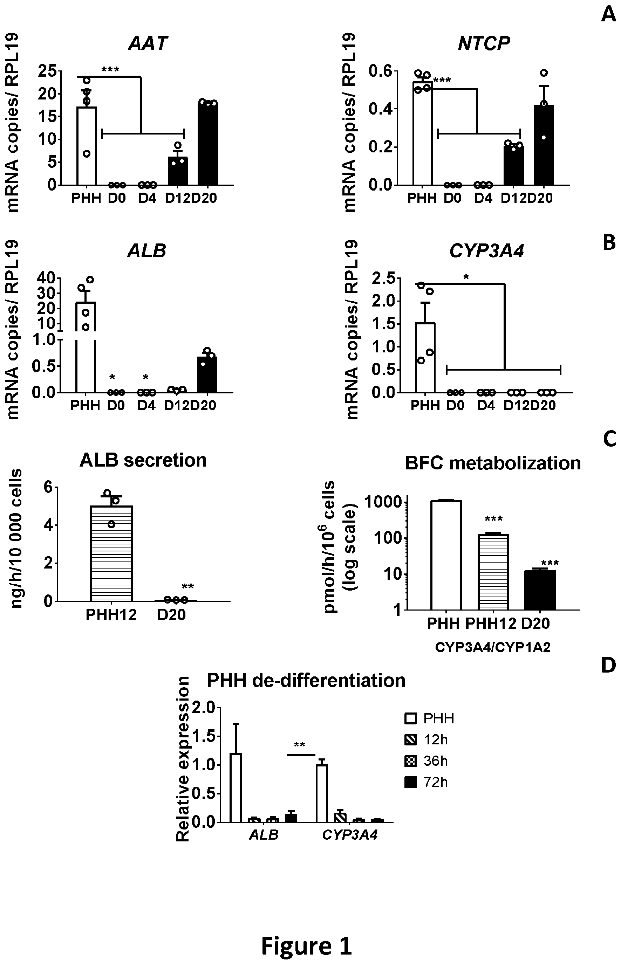

In order to evaluate and improve CYP450 function in PSC-derived HLCs, we differentiated human embryonic stem cells (hESCs; H9 cells) to the hepatic lineage and compared the transcriptional profile of HLCs with that of cryopreserved PHHs from 4 donors. By d20 hepatocyte specific transcripts, such as a1-antitrypsin (*AAT)* and Na+-taurocholate co-transporting polypeptide (*NTCP),* reached levels near those in PHHs (hepatocytes isolated by laser capture microscopy from cryopreserved liver biopsies of 3 different donors; termed hereafter "Fresh PHHs"). (Figure **1A**). However, consistent with our and other previous reports, transcript levels for mature hepatocyte genes, such as albumin (*ALB*) or cytochrome P450 (*CYP)3A4,* one of the key phase 1 drug metabolizing enzymes in the human liver, remained significantly lower than in Fresh PHHs (Figure **1B**). Consistently, PSC-progeny secreted significantly less albumin and had significantly lower CYP3A4 activity compared with freshly thawed PHHs and PHHs plated for 12h in collagen-coated wells (termed hereafter "PHH12") (Figure **1C****).** As also demonstrated by others, PHHs quickly lose their mature features, including ALB and CYP3A4 expression, thus indicating that current culture conditions do not allow for the induction or maintenance of mature hepatic function (Figure **1D**).

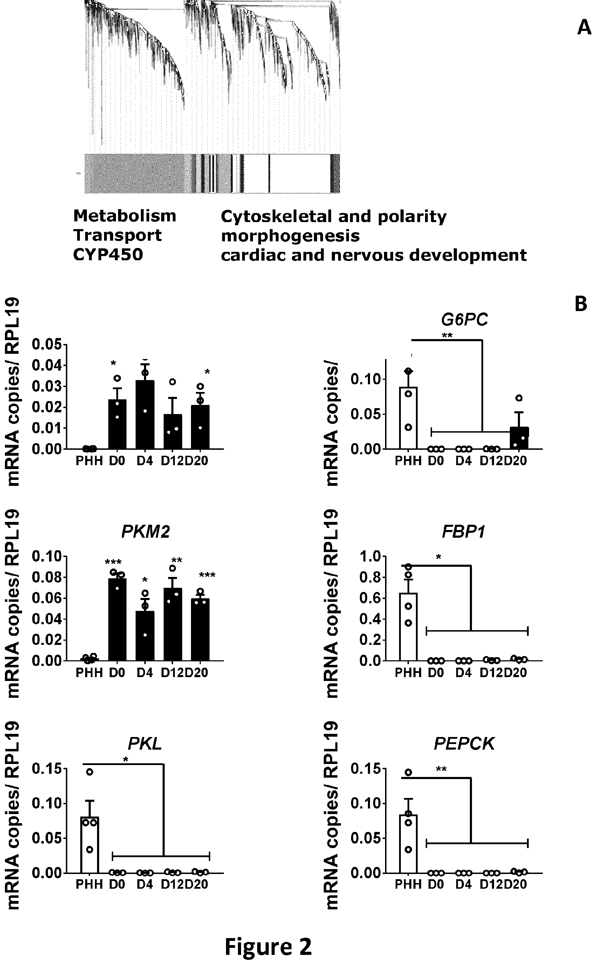

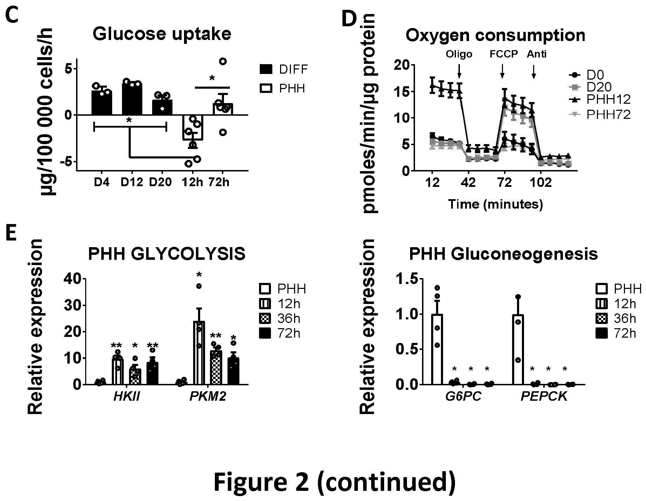

To gain molecular insights in the poor CYP450 expression of HLCs, we performed a weighted Correlation Network Analysis (WGCNA) on in house and available transcriptome data. We identified two major gene clusters that differ significantly between HLCs and PHHs. As already described by others, one cluster consisted of genes involved in developmental pathways and genes linked to the cytoskeleton, the latter likely the result of culture in stiff culture dishes. Unexpectedly, CYP450 genes were found in a separate cluster together with regulators of cellular metabolism, including gluconeogenesis, mitochondrial metabolism and amino acid catabolism (Figure **2A**). This finding formed the basis for the novel hypothesis that an immature cellular metabolism is the prime reason for low CYP450 expression, and only to a lesser extent the immature phenotype due to culture conditions, as previously proposed. PSCs are known to be highly dependent on glycolysis for energy production, whereas hepatocytes represent the main gluconeogenic cell type in the body. However, transcripts for glycolytic genes, such as *HKII* and *PKM2,* did not decrease during hepatic differentiation from PSCs, while genes important for gluconeogenesis (glucose 6 phosphatase (*G6PC*), fructose-bisphosphatase 1 *(FBP1)* and phosphoenolpyruvate carboxykinase (*PEPCK*)) were not induced. Furthermore we did not observe a switch towards the liver specific pyruvate kinase (*PK*) variant, *PKL,* while the important glycolytic enzyme *PKM2,* remained highly expressed (Figure **2B**). Consistent with the gene expression, we also observed a continued high rate of glucose consumption during HLC differentiation, while PHH12 produced glucose (Figure **2C**). In accordance, measurements of oxygen consumption rates (OCR) confirmed a lower basal mitochondrial activity in PSCs and HLCs compared to PHH12. (Figure **2D**).Thus, PSC-HLCs do not only express significantly lower levels of mature hepatic markers (e.g. ALB and CYP3A4) but also possess an immature cell metabolism (glycolysis, no gluconeogenesis, and low mitochondrial activity) compared with PHHs; a phenotype also seen when PHHs are cultured *in vitro.*

### Overexpression of hepatocyte transcription factors induces functional and metabolic maturation.

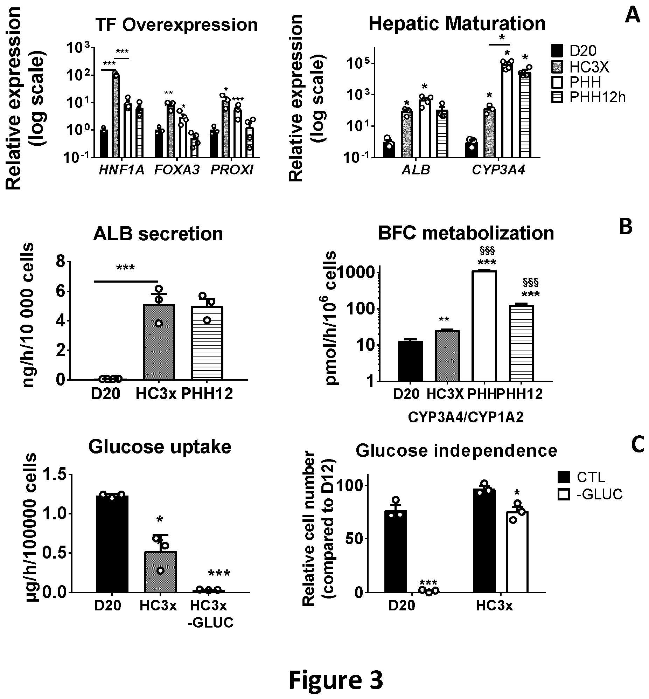

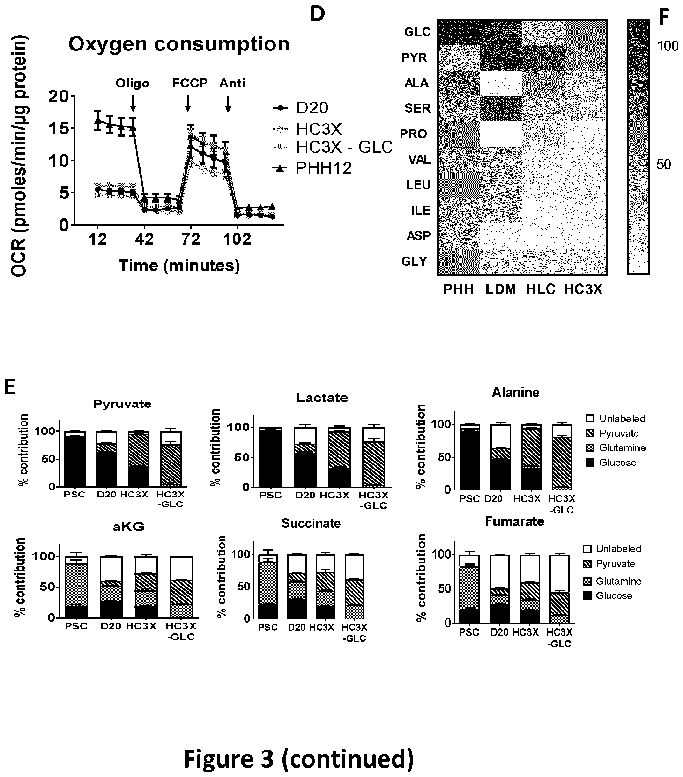

Overexpression of hepatic TFs has been shown to enhance maturation of HLCs to some extent^{10,17} According to our hypothesised link between cellular metabolism and CYP450 enzymes, more mature cells would therefore need to also show a rewiring of metabolic pathways. To overexpress missing transcription factors, we performed recombinase mediated cassette exchange (RMCE) in the H9-ESC line containing an exchangeable cassette in the adeno-associated virus integration Site 1 (*AAVS1)* locus¹⁸. We recombined a multicistronic sequence containing the coding domain for *HNF1A,* forkhead box *(FOX)A3* and Prospero homeobox protein *(PROX1)* under the control of a TET-on promoter system (termed HC3X cells). Doxycycline-mediated induction of *HNF1A, FOXA3* and *PROX1* from day 4 until day 20 induced expression of the three TFs at levels near those of PHHs. This was also associated with an induction in both *ALB* and *CYP3A4* expression. Transcript levels of *ALB* now reached those of PHH12 and *CYP3A4* expression was increased 50-fold compared to HLCs (Figure **3A**). Although albumin secretion was found to be equal to PHH12, the metabolisation rate of 7-benzyloxy-4-trifluoromethylcoumarin (BFC) was still very low when compared to PHHs (Figure **3B**). Interestingly, overexpression of the three hepatic TFs was associated with partial metabolic maturation. Indeed, glucose consumption was reduced and HC3X-progeny could be cultured without glucose (Figure **3C**). However, no glucose secretion or increased OCR was observed, indicating that this combination of TFs could not fully rewire hepatic metabolism nor induce mature hepatocyte CYP450 expression (Figure **3C** **and** **3D**).

As HC3X-progeny could be cultured in the absence of glucose, we next evaluated which nutrients fueled the central carbon metabolism of PSCs, HLCs and HC3X-progeny *in vitro.* We treated the different cell populations with ¹³C labeled tracers for glucose, glutamine and pyruvate, and measured their contribution to glycolytic (pyruvate, lactate and alanine) and tricarboxylic acid cycle (TCA) (alpha-ketoglutarate, succinate and fumarate) metabolites. Undifferentiated PSCs and HLCs displayed a phenotype characterized by high glucose-derived ¹³C labeling of glycolytic metabolites and high glutamine-derived ¹³C labeling of TCA metabolites. In HC3X-progeny, significantly more pyruvate-derived ¹³C labeling was seen in all intermediates, which was even more pronounced upon glucose withdrawal (Figure **3E**). Yet, ¹³C labeled glucose, glutamine and pyruvate did not account for the entirety of the produced metabolites in both HLCs and HC3X cells, suggesting that hepatic progeny consumed additional carbon sources. Indeed, when measuring nutrient uptake we found that amino acids were highly depleted in all hepatic cells cultured *in vitro.* This indicates that an exhaustion of amino acids from the culture medium of PSC- and HC3X-hepatic progeny after 24h might cause their glucose / pyruvate dependence.

### Amino acids induce gluconeogenesis and mitochondrial activity in a concentration dependent manner.

We hypothesized that amino acids are the limiting factor to induce gluconeogenesis and mitochondrial activity. Therefore, we differentiated PSC/HC3X cells between d12-d20 either in Williams E based Hepatocyte Maintenance Medium (782 mg/l Amino acids), or in liver differentiation medium (LDM), supplemented with increasing concentrations of Amino acids (LDM= 214.28 mg/l, AA1=649.62mg/l, AA2= 1955.62mg/l and in AA3 =3696.97 mg/l total Amino acids). The composition of the amino acids in these media is shown in the below table.

**Table1. Concentrations of amino acids in tested media**

| | **LDM (mg/l)** | **WE-hepatocyte medium (mg/l)** | **AA1 (mg/l)** | **AA2 (mg/l)** | **AA3 (mg/l)** | **AA3 + GLY (mg/l)** |
|---|---|---|---|---|---|---|
| Glycine | 3,01 | 50 | 17,95 | 62,77 | 122,53 | 20122,53 |
| L-Alanine | 3,56 | 90 | 21,29 | 74,478 | 145,39 | 145,39 |
| L-Asparagine | 60 | 20 | 85,2 | 160,8 | 261,6 | 261,6 |
| L-Aspartic acid | 5,2 | 30 | 31,69 | 111,18 | 217,16 | 217,16 |
| L-Glutamic Acid | | 45 | 34,88 | 122,75 | 239,90 | 239,90 |
| L-Proline | 2,28 | 30 | 25,23 | 94,09 | 185,91 | 185,91 |
| L-Serine | 12,42 | 10 | 33,17 | 95,43 | 178,43 | 178,43 |
| L-Arginine HCl | 25,25 | 50 | 25,25 | 88,19 | 277,04 | 277,04 |
| L-Cystine | 14 | 40 | 25,86 | 61,44 | 108,88 | 108,88 |
| L-Histidine HCl-H₂O | 8,02 | 15 | 28,94 | 91,70 | 175,38 | 175,38 |
| L-Isoleucine | 5,26 | 50 | 31,41 | 109,85 | 214,44 | 214,44 |
| L-Leucine | 15,66 | 75 | 41,70 | 119,83 | 224,00 | 224,00 |
| L-Lysine hydrochloride | 15,27 | 87 | 51,37 | 159,66 | 304,05 | 304,05 |
| L-Methionine | 1,76 | 15 | 9,29 | 31,88 | 62,019 | 62,02 |
| L-Phenylalanine | 1,96 | 25 | 18,44 | 67,88 | 133,80 | 133,80 |
| L-Threonine | 14,05 | 40 | 37,71 | 108,69 | 203,33 | 203,33 |
| L-Tryptophan | 2,40 | 10 | 7,48 | 22,71 | 43,016 | 43,016 |
| L-Tyrosine | 4,47 | 50 | 22,42 | 76,29 | 148,11 | 148,11 |
| L-Valine | 14,05 | 50 | 37,31 | 107,09 | 200,13 | 200,13 |
| **Total Amount** | **214,28** | **782** | **649,62** | **1955,6 2** | **3696,9 7** | **23696,97** |

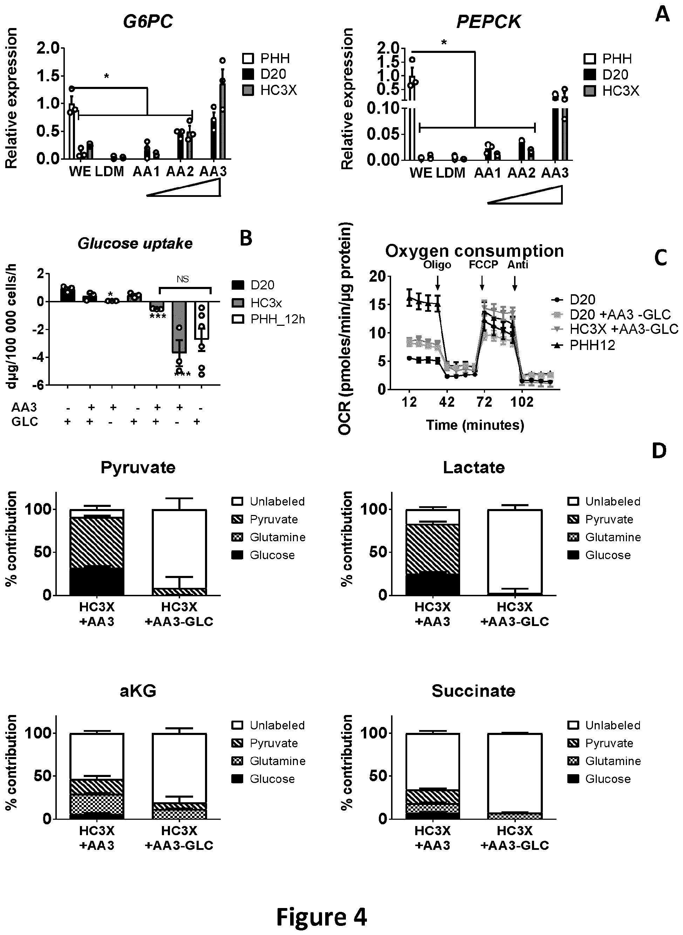

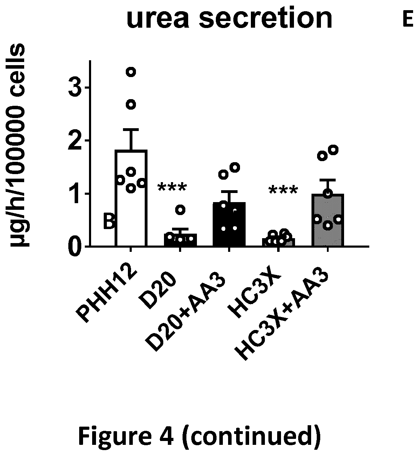

We found an amino acid concentration dependent increase in transcripts for *G6PC* and *PEPCK* in both PSC and HC3X-progeny (Figure **4A**).When differentiated in the presence of high levels of AA's (AA3), HC3X-progeny became not only glucose independent, but also secreted glucose (Figure **4B**). Also, amino acid addition and glucose removal doubled PSC- and HC3X-progeny mitochondrial function as measured by OCR (Figure 4C).¹³C tracing experiments demonstrated a massive dilution in all measured metabolic intermediates by unlabelled carbons (Figure **4D**), whereas ureum secretion was found to be induced to the same level as found in PHHs (Figure **4E**).

### Increase of amino acid concentration induces mature hepatic drug metabolization in PSC derived HLCs

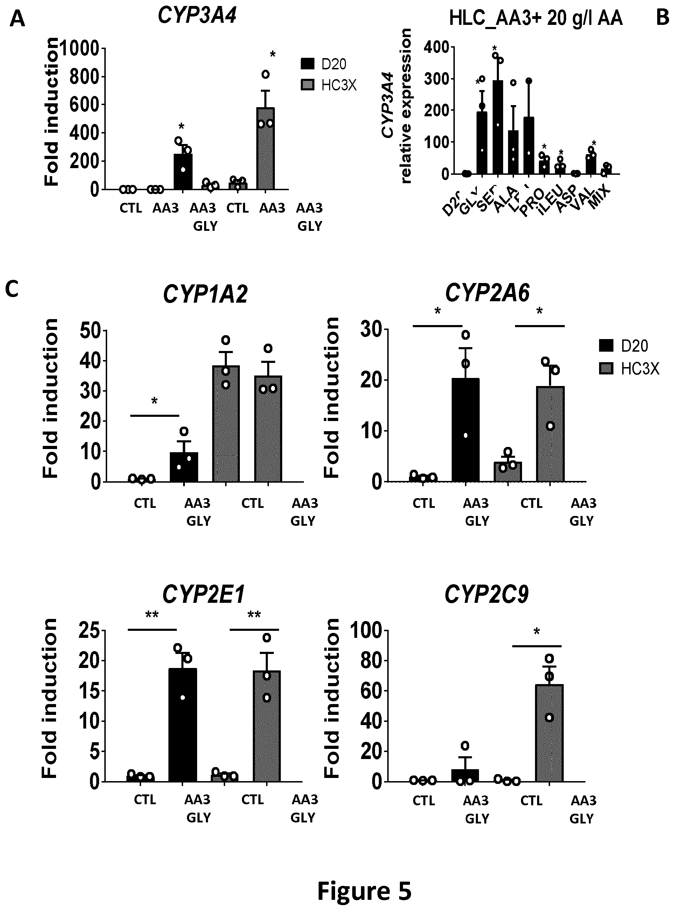

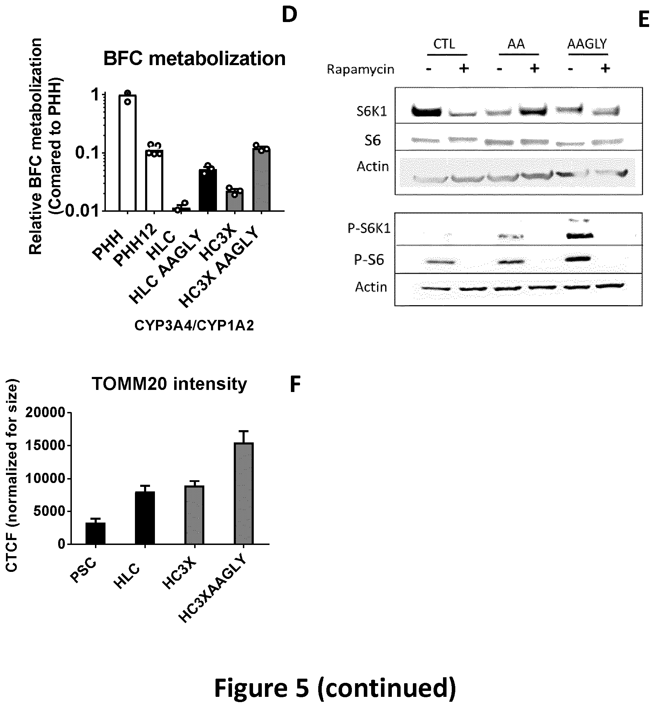

As the WGCNA of deregulated pathways in HLCs revealed a clear co-correlation between central carbon metabolism, AA catabolism and CYP450 gene expression (Figure **2A**), we next tested the effect of AA supplementation on CYP450 enzyme expression and function. AAs induced the expression of CYP3A4, but only at concentrations even higher than those necessary for the induction of gluconeogenesis. As shown in Figure **5A****,** CYP3A4 levels increased 200-800 fold when HLCs or HC3X cells were differentiated in AA3 further supplemented with 20g/l of glycine (AAGLY). This effect was not glycine-specific, as a similar induction was observed upon addition of serine, alanine, leucine, proline, isoleucine and valine, or a mixture of all of the above AAs, that together constituted 20 g/l (Figure **5B**). Besides from CYP3A4, also other CYP450 isoforms such as CYP2A6, CYP1A2, CYP2C9 and CYP2E1 were induced in AAGLY medium (Figure **5C**). This induction translated in functional improvement as CYP3A4-dependent metabolisation of BFC now became equal to that of PHH12, i.e. 5-10% of PHHs (Figure **5D**). Importantly, this induction was only found at very high AA concentrations, as was the activation of the cellular sensor for amino acid concentrations mammalian target of rapamycin complex (mTORC). Western blot analysis for phosphorylated p70S6 (S6) and p70S6 kinase (S6K) demonstrated that the mTORC pathway was only activated in HC3X cells cultured in the presence of very high levels of AAs (AAGLY) (Figure **5E**). These results indicate that indeed these very high concentrations are needed to induce intracellular signalling pathways, including mTORC, to increase CYP450 expression. Besides from CYP450 function, we also found mitochondrial mass, a critical feature of mature hepatic function, to be increased only under these conditions.

### High amino acid concentrations allow for CYP450 induction over time to levels that are equivalent to PHHs

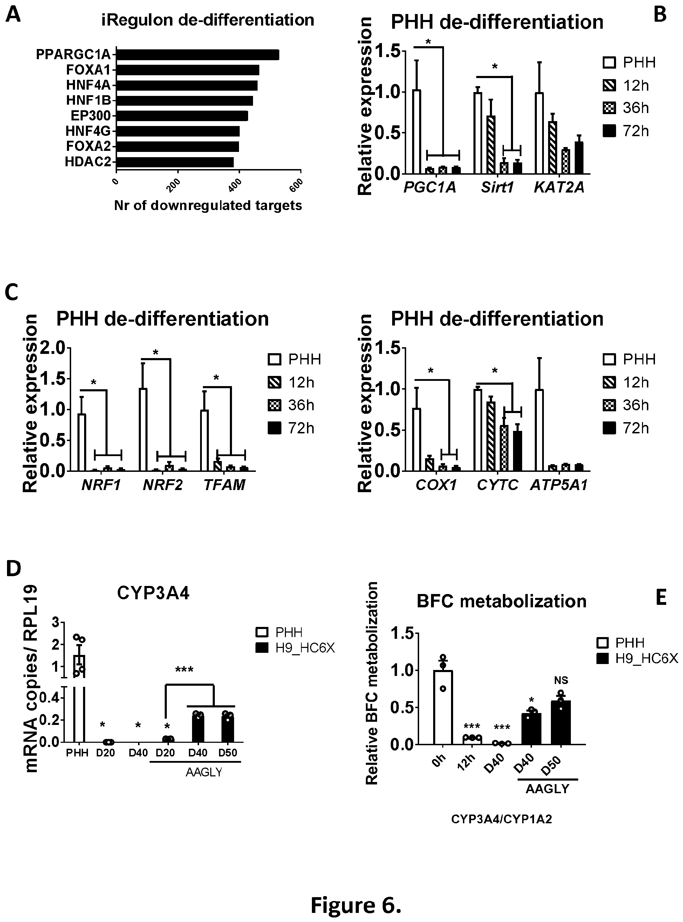

As we uncovered a clear correlation between AA concentrations, hepatic metabolism and mature hepatic CYP450 function, we next tested whether an overexpression of metabolic regulators might induce maturation even in the absence of AAs. Interestingly, iRegulon analysis¹⁹ of the transcriptome of fresh and plated PHH. identified the metabolic master regulator peroxisome proliferator-activated receptor gamma coactivator (PGC)-1α (PPARGC1A) as the TF with the most deregulated targets (Figure **6A**). We indeed, validated that culture conditions decrease both the expression of PGC-1a and its upstream activator Sirtuin (SIRT)1, but not its upstream inhibitor KAT2A (Figure **6B**). Furthermore, also several downstream targets of PGC1α, including nuclear respiratory factor (NRF)1, NRF2, transcription factor A, mitochondrial (TFAM), and the mitochondrial proteins COX1, CYTC and ATP5A1 were all decreased upon plating of PHHs (Figure **6C**). We therefore next tested whether further activation of mitochondrial metabolism through the overexpression of PGC-1a would allow for a further maturation of PSC-derived cells in AAGLY-supplemented media. We thus generated a PSC line containing an inducible cassette for the 3 TFs already present in the HC3X line, PGC-1a as well as its post-transcriptional regulator SIRT1 and a constitutively active variant of 5' AMP-activated protein kinase (AMPK; an inducer of PGC-1a and SIRT1) (termed HC6X cells). Importantly, overexpression of these regulators did not induce any CYP450 function as such, but did allow for further maturation over time when combined with supplementation of high levels of AAs. Indeed, CYP3A4 expression was found to be the same as freshly isolated PHHs after 50 days of differentiation (Fiugure **6D**), as was the metabolization rate of BFC (Figure **6E**).

### The combination of transcript and nutrient optimization allows for the prediction of hepatocyte specific toxicity in a high throughput format.

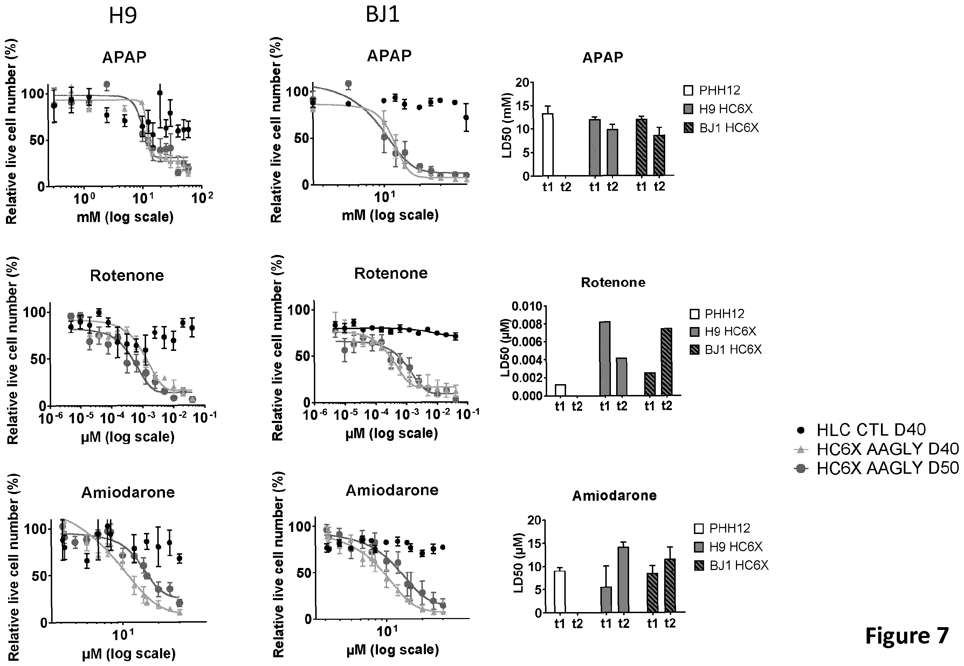

As the inducible overexpression of hepatic and metabolic regulators combined with nutrient optimization induced a gluconeogenic profile, mitochondrial activity, and CYP450 gene expression and function near levels of plated PHHs, and this stable for over 10 days, we tested if HC6X and HC3X progeny were suitable to predict drug induced liver toxicity. We cultured HC6X cells(both in an H9 background and in an ) BJ1-iPSc background in 384 well format in AAGLY-supplemented medium. We tested their sensitivity to two drugs that require cytochrome P450 dependent metabolisation; amiodarone (metabolised by CYP1A2, 2C19, 2C9, 2D6, 3A4) and acetaminophen (APAP; metabolised by CYP2E1, 1A2, 3A4). In addition, as PHHs are known to be highly sensitive to mitochondrial toxins, and AA supplementation was found to increase both mitochondrial mass and activity, we tested if the cells would accurately predict toxicity to the known mitotoxin, rotenone. Cells were exposed to 14 different concentrations of drugs using 2-fold dilutions starting at 30 mM for amiodarone, 60 mM APAP and 0.04 µM rotenone and this for 24h, and then stained with Hoechst and the live-dead stain Draq7 to enumerate living cells using an automated high-throughput Operetta High-Content Imaging System. Although no toxicity was seen for HC6X progeny cultured in basal medium (CTL), AAGLY medium resulted in sigmoidal kill curves that were similar to PHHs, with LD50 values similar to those of PHHs, and in line with previously published PHH-based toxicity studies42,5(Figure 6H and supplementary table ST3). Most importantly, both cell models were found to be stable, as similar drug toxicity was seen after a further 10 days of culture (Figure 7A).

### Example 2: Media improves function of PSC-derived hepatocytes

In the previous example we showed that the supplementation of high levels of amino acids to the culture medium of PSC-derived hepatocytes induced both metabolic and functional maturation to levels found in PHHs. Furthermore, we showed that only under these conditions, the cells could be used for the screening of hepat- and mito-toxic compounds in relevant concentrations, and for a stable duration. Since this finding is of high interst for the pharmaceutical industry, we next tested whether the same media optimization might also result in maturation of commonly used hepatocellular carcinoma cell lines, that are now commonly used in industry.

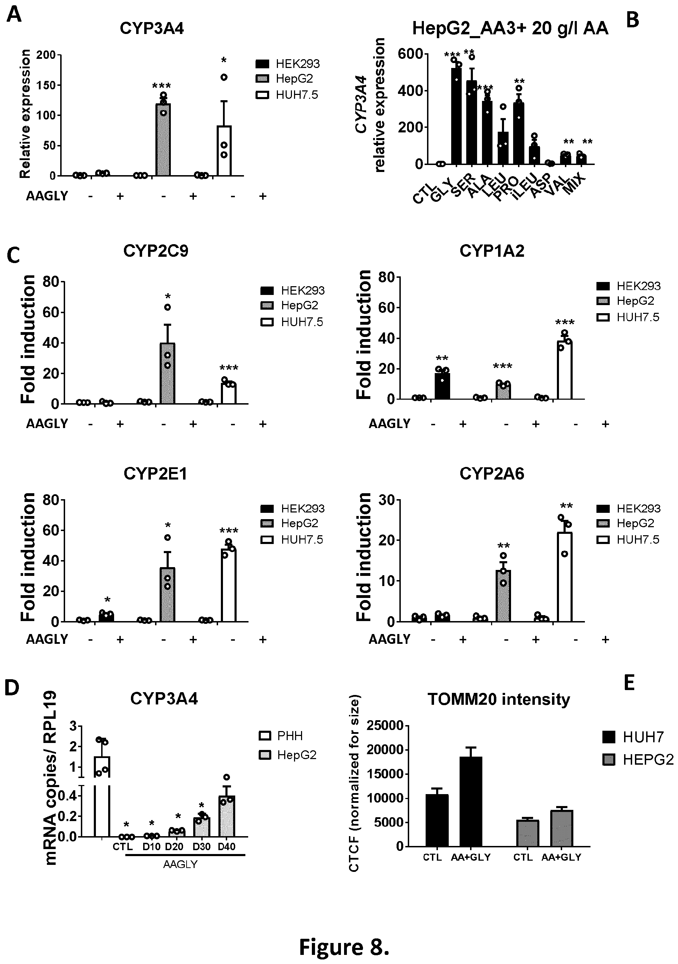

Indeed, supplementation of the AAG-composition to standard culture media (composition as shown in material and methods) of either hepG2 or HUH7 hepatoma cell lines greatly induced the expression of CYP3A4 (Figure **8A**) and other CYP450 isoforms (Figure 8C) after 7 days of incubation. Again this induction was not glycine- , but rather concentration specific, as glycine could be exchanged with any of the testes other amino acids (Figure **8B**). As expected, hepatocyte-specific CYP450 enzymes were not induced in the embryonic kidney cell line HEK293. However, CYP1A2 expression was induced in this cell line. As this isoform has a role in kidney cells ²⁰, these results might indicate that AA supplementation might improve xenobiotic and or overall metabolism of in vitro cultured cells other than hepatocytes.

As we observed wit hPSC-*derived hepatocytes, prolonged culture of HepG2 cells in AAGLY supplemented medium further increased maturation as CYP3A4 expression as found to be equal to PHHs after 30 days (Figure **8D**). Last, both in HUH7 and HepG2 cells, AAGLY supplementation resulted in a significant induction of mitochondrial mass, as measured by the intensity of TOM20 staining (Figure **8E**).

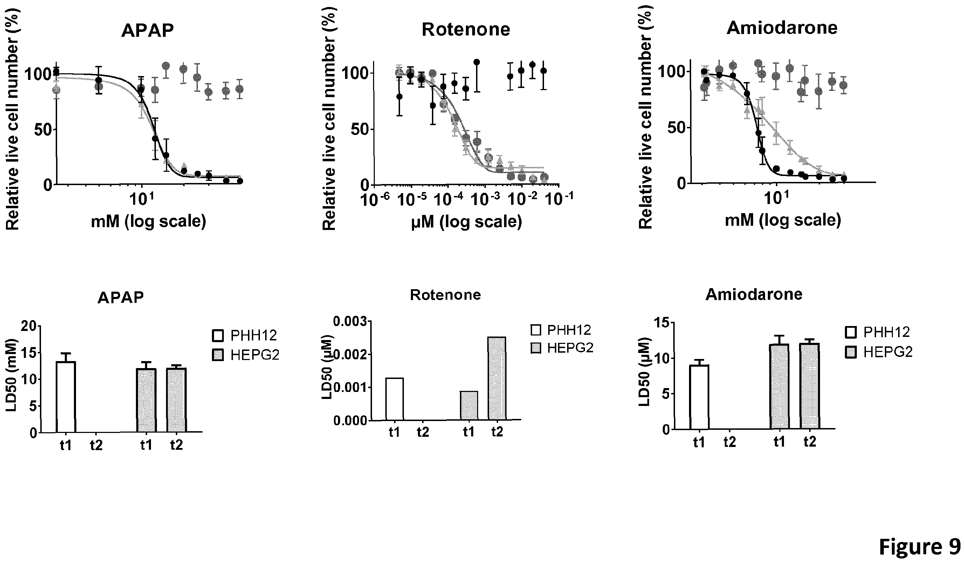

In order to test whether also hepatic cell lines are suitable for toxicity screenings after medium optimization, we cultured HepG2 cells for 30 days in a 384 well format in the AAGLy-supplemented medium and exposed them for 24h to the same drugs as utilized to test the PSC-derived cells. Indeed, also HepG2 were able to predict toxicity of APAP, rotenone and amiodarone, in concentrations that are consistent with data obtained for PHHs, and in a stable manner. Importantly, HepG2 cells grown in standard media were not able to pick up toxicity, thus proving that AA-supplementation is also a valuable tool to induce maturation in hepatic cell lines (Figure **9**).

### Reference

### Example 3: the media also induces functional improvement in non-hepatic cells

Data from several studies indicate that the correlation between an immature cellular phenotype and a dysregulated nutrient utilization in vitro is not a liver-specific phenomenon, but that most cell types display the same problem. Differentiation protocols aimed at generating functional cardiomyocytes from PSCs have been found to generate cells that structurally resemble the phenotype observed in the embryonic hearth tube ²¹ and also transcriptionally resemble fetal heart tissue ²² Like hepatocytes, also myocardial cells display very high levels of mitochondrial activity, a large and elaborated mitochondrial network and a high dependency on OXPHOS²³. By contrast, and as we observed for PSC-hepatocytes, both fetal and PSC-derived cardiomyocytes use glycolysis²⁴, even if mitochondrial abundance increases following prolonged culture²⁵. However, cardiomyocytes appear to be sufficiently mature to become glucose independent, as a replacement of glucose by lactate was shown to be an efficient way to purify cardiomyocytes²⁶. This indicates that, as for hepatocytes, cardiac maturation might be dependent on nutritional signals currently missing in the culture medium. Indeed when we differentiate PSCs towards the cardiac lineage in the presence of high levels of AAs (AAGLY), we also observe, as we did for the hepatic lineage;
1. An induction in PGC-1a and mitochondrial proteins
2. Functional maturation in a time dependent manner

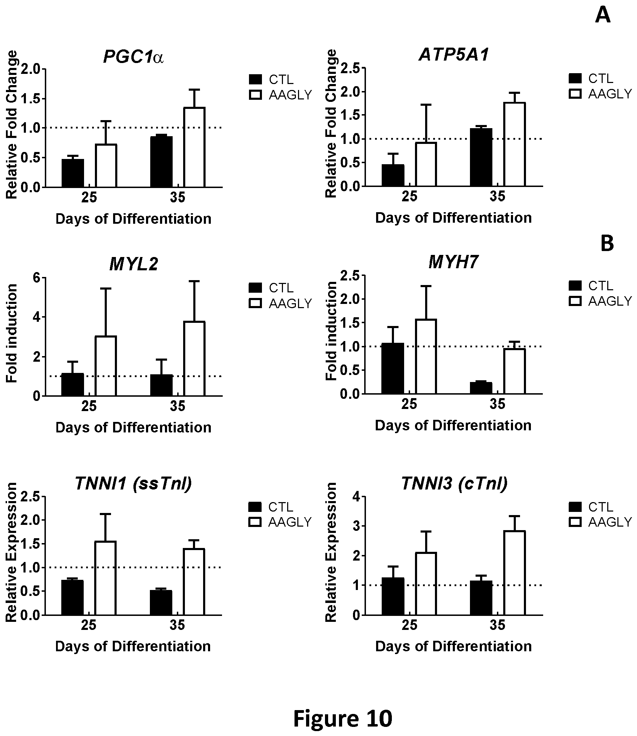

Indeed, as shown in figure **10A****,** also in PSC-derived cardiomyocytes, PGC1A and the mitochondrial protein ATP5A1 are induced upon culture in AAGLY-medium. Interestingly, also the expression for the mature cardiac genes Myosin light chain (MYL)2 and 7 and Troponin (TNNI)1 and 3 are induced by AAGLY supplementation and in a time dependent manner (Figure **10B**). This would indicate that also for cardiomyocytes, AA-supplementation to levels far beyond commonly used represents a manner to allow for further differentiation and acquirement of a mature phenotype.

### DISCUSSION

The major finding of this study is the observation that the capacity of hepatic cells to metabolise drugs is critically dependent on their profile of nutrient utilization. Second, we demonstrate that AA concentrations, that were found to be limiting in currently used media formulations, are able to actively influence the expression of both metabolic regulators, mitochondrial genes and CYP450 enzymes. The consequence of this is that PSC-derived hepatic progeny and hepatic cell lines cultured in the optimized nutrient microenvironment acquire the capability to metabolise drugs at levels similar to those found in PHHs. Thus, this study demonstrates that nutrient engineering can be used to steer PSC differentiation to generate more mature cells.

In the trail of cancer metabolism, investigators have only recently started to assess metabolic pathways in the context of stem cell maintenance and differentiation. Elevated glycolysis has been shown to be an essential part of stemness, and glycolytic enzymes are activated through core components of the pluripotency network²⁷. Hepatocytes on the other hand, are known to possess a highly elaborate mitochondrial network²⁸ and mitochondrial dysfunction has been implicated in several liver related diseases²⁹. In the context of HLC differentiation, two groups recently described the essential requirement of induction of mitochondrial biogenesis and activity²⁸,³⁰. These results are in accordance with our identification of a crucial link between mitochondrial metabolism and CYP450 activity.

Metabolic engineering through the identification of metabolites and nutrients that steer (stem) cell metabolism and fate, has only recently been proposed as a valid strategy to induce differentiation, next to standard treatments such as growth factors, ECM engineering and co-culture studies³¹. For instance, addition of oxidized metabolites can drive PSC differentiation towards the neuronal lineage¹⁶, while a switch towards a lactate fuelled metabolism allows to effectively purify cardiomyocytes from differentiation protocols²⁶. Furthermore, addition of extracellular α-ketoglutarate promotes reprogramming through the activation of DNA demethylation¹⁵, whereas removal of methionine is sufficient to induce differentiation³².

Only a few studies have investigated the metabolome of primary hepatocytes. In one study, it was suggested that the overall metabolite concentrations are much higher in adult than fetal cultured hepatocytes³³. Another study demonstrated that cultured PHHs are in an extremely starved state, suggesting the need for nutrient optimisation³⁴. Through analysis of carbon fluxes fuelling cellular metabolism, we found that currently used medium compositions are the reason for the observed dependency on glycolysis of PSC-derived HLCs. Although previous studies identified that amino acids are important for survival, proliferation and protein synthesis of PHHs^{35,36}, we here demonstrate that increasing amino acid concentrations to levels 30-fold above that of typical hepatocyte media, actively induces a metabolic switch (from glycolysis to mitochondrial metabolism and gluconeogenesis) as well as CYP450 enzyme expression Importantly, increasing AA concentrations has been implicated in the maintenance of albumin secretion, DNA replication, protein synthesis and survival of PHHs, but has never been linked with mitochondrial function or CYP450 expression (table 2). We show that even under concentrations found in the most rich media, i.e. Leibovitz L-15, CYP450 function and mTORC signalling is not induced.

**Table 2. Overview of AA concentrations present in typical hepatocyte culture media**

| **Reference** | **Cell model** | **Basal media** | **Total AA concentration** |
|---|---|---|---|
| ATCC | Cell line culture | DMEM supplemented with glutamine | 1.1 g/l + 0.5 g/l glutamine |
| Sigma | Cell line culture | DMEM with glutamine and non-essential amino acids | 1.185 g/l + 0.5 g/l glutamine |
| 37 | Rat primary hepatocytes | Ham's F12:Dulbecco's supplemented with arginine, tyrosine, methionine, proline and glycine to levels that exceed 10 times that of rat arterial plasma | 2.94 g/l |
| 38 | PSC differentiation | CMRL/Hepatozyme (Invitrogen) | 0.944 g/l |
| 39 | Primary hepatocytes | Williams' Medium E | 0.782 g/l |
| 40 | PSC differentiation | Hepatocyte Basal Medium (Lonza CC-3199) | 0.782 g/l |
| 41 | PSC differentiation | Williams' E medium | 0.782 g/l |
| 42 | PSC differentiation | DMEM/F12 with 1 mmol/L nonessential amino acids | 1.189 g/l |
| 5 | Primary hepatocytes | William's E medium | 0.782 g/l |
| 43 | PSC differentiation | RPMI B27 | 0.86808 g/l |
| 12 | Primary hepatocytes | William's E medium with 2 mM of glutamine | 1.075 g/l |
| 44 | Primary hepatocytes | William's E medium with 2 mM of glutamine | 1.075 g/l |
| 45 | Primary hepatocytes | Amino acid-rich medium (Leibovitz L-15) medium | 3.3 g/6 |
| **Boon et al** | PSC differentiation Cell line maturation | **AA3-glycine medium** | **23.696 g/l** |

Although limited data is available very high concentrations of aspartate, taurine, glutamine, glutamate, glycine and alanine, have been reported in the hematopoietic stem cell marrow niche⁴⁶ and the hepatocyte niche in vivo⁴⁷. The latter is consistent with the notion that the liver is the major detoxifying organ for ammonia, and with the fact that AAs fuel glucose, urea and protein production in a concentration dependent manner in vivo⁴⁸. It has also been shown that AAs, much higher in portal blood than peripheral blood, activate hepatic protein synthesis in an mTORC1-dependent manner that is independent of hormonal signaling⁴⁹. In accordance, we here provide data that the AA concentrations dictate the hepatic phenotype.

We, however, also found that nutrient engineering alone is not sufficient to induce maturation of PSC-derived HLCs. We demonstrated that this also requires the overexpression of transcriptional regulators that redirect hepatic metabolism and induce mitochondrial biogenesis through PGC-1a. Therefore, we exposed an interplay between transcriptional signalling pathways and nutrient levels to fully determine hepatic fate. Nevertheless, hepatocellular carcinoma lines such as HepG2, do not require TF overexpression for AA-mediated maturation. Interestingly, HepG2 cells have been shown to be able to switch to some extend from glycolysis to a more mitochondrial metabolism through the replacement of glucose by galactose⁵⁰. HepG2 cells have been described as the most differentiated hepatic cell line available⁵¹ and this cell line also performs the best in identifying hepatotoxins in screening models⁵². This would indicate that only cells with a high base-level of maturity can respond to the AA supplementation. This is also of very high practical importance as AA supplementation can be easily implemented in already established screening systems that rely on these cell lines.

Last and of high practical importance, we were demonstrated that drug metabolisation capacity and drug toxicity susceptibility of the metabolically (and genome) engineered PSC- and HepG2 based model systems approach those of the current gold standard systems. Both models now allow studying drug metabolisation; and as a consequence also assessment of toxicity caused by drugs that require hepatic metabolisation, such as APAP and amiodarone with sensitivity approaching PHHs⁵³,⁵⁴. Moreover, due to the increased mitochondrial activity of HC6X and HepG2 cells in AAGLY medium, these cell models enable assessing mitochondrial toxins, such as rotenone, known to be highly lethal for hepatocytes⁵⁰. Finally, and in contrast to PHHs cultured in classical 2D culture conditions, we also show that both cell models are highly stable and allow drug toxicity and drug metabolisation studies for at least 10 days, such that they might be suitable for repeat toxicity studies. Also, we show that the same media composition can induce kidney-specific CYP450 function in cells with a kidney origin, and can induce maturation in PSC-derived cardiomyocytes. These results indicate that our invention represents a nutrient engineering strategy to manipulate mitochondrial mass and activity and cellular behaviour in a wide range of cell systems and applications.

In conclusion, this study highlights the need for investigating nutrients as a potential novel source to guide PSC-fate and cellular behaviour in cell models. The optimisation described creates significantly improved and long(er)-term stable hepatocyte models for studying hepatocyte biology, drug metabolisation, and the stable nature of the models should allow for repeated dose drug toxicity studies in vitro. Furthermore, this optimization will also have consequences in other organ systems such as the hearth and kidney.

The methods have the present invention have been exemplified with media typically comprising 20mg/ml glycine. Experiments with 10 and 15 mg/ml glycine, equally provide results which are significantly better than control media with a mixture of amino acids at 3mg/ml medium.

### MATERIALS AND METHODS

### hESC Differentiation to the Hepatocyte Lineage

The hESC line H9 (WA09) was purchased from WiCell Research Institute (Madison, 15 WI) and kept according to supplier's instructions as feeder free cultures on matrigel (BD biosciences) coated plates in Essential 8 (or Essential 8 Flex) (Thermo fisher). H9 cells were differentiated towards the hepatocyte lineage as described by our group before ⁹, with some optimizations. Briefly H9 cells were made single cell using accutase and plated on matrigel-coated plates at ± 8.75 x 10⁴ cells/cm² in mTeSR medium. When cells reached 70-80% confluency differentiation was started using the previously described cytokine regimes and was stopped after 20 days of differentiation. All cytokines were purchased from Peprotech (NJ). Differentiation medium was supplemented with 0.6% dimethylsulfoxide (DMSO) during the first 12 days of the culture and with 2% DMSO during the last 8 days of differentiation.

### Primary cells and cell lines

As positive control for hepatic expression and function, 2 donors (C304 (64 year, male) and C303 (52 year, female) of crypopreserved and platable primary human hepatocytes were bought from Corning Biosciences (Corning^{®} Gentest^{™} Plateable Human CryoHepatocytes, Metabolism-Qualified, ≥5 million cells (Product #454543). Furthermore, two additional donors were obtained from the Hepatocytes and Hepatic Stem Cells bank from the Cliniques Universitaires Saint-Luc in Brussels; donor F125 (male, 62 years of age) and donor F110 (Female, 26 years of age). All 4 donors were used for functional assessment, while seahorse and toxicity experiments were performed on donor C303 and F125. Primary cells were thawed using the Corning^{®} Gentest^{™} High Viability Cryo Hepatocyte Recovery Kit (Corning) and plated on collagen I coated plates. Collection of clinical samples: Human liver samples used for metabolomics were obtained from the Laboratory of Hepatology (UZ Leuven - KU Leuven, Belgium), respecting patient's rights and collected upon ethical approval of local authorities.

### Recombinase mediated cassette exchange

The master cell line suitable for RMCE was generated using zinc-finger mediated integration of an FRT-flanked donor cassette into the *AAVS1* locus as described previously¹⁸. RMCE was performed by nucleofection of the master cell line with a donor vector and the FLPe-expressing vector. Nucleofection was done on 3 million cells obtained after accutase treatment using the hESC Nucleofector Solution Kit 2 (Amaxa) and program F16 using an Amaxa nucleoporator. Cells were plated on puromycin-resistant mouse embryonic feeders (MEFs) (Stem Cell technologies, Puromycin-Resistant Mouse Embryonic Fibroblasts, Day E13.5) in hESC medium as described⁵⁵. Donor plasmids were generated through Gibson assembly (NEB) of PCR amplified ORFs of the desired genes. Plasmids were then evaluated by digestion and Sanger sequencing. The constitutively active AMPK construct was bought from Addgene (plasmid nr. 60127) and generated by the lab of Prof. Morris (University of Pennsylvania).

### Media optimization and composition

Differentiation of PSCs (and genetically modified PSCs) was performed in liver differentiation medium (LDM) as previously described ⁹ (The composition is given in supplementary table **ST6**). ¹³C labeled carbon sources were added to medium made from powder of Dulbecco's Modified Eagle's Medium (DMEM) without glucose, L-glutamine, phenol red, sodium pyruvate and sodium bicarbonate. As MCDB also contains glucose the MCDB component of LDM was replaced by DMEM.

AA supplementation was accomplished through the addition of MEM Non-Essential Amino Acids Solution (100X) and MEM Amino Acids Solution (50X) (Thermo Scientific) to the culture medium. For AA1, AA2 and AA3 medium we added respectively 2ml, 8ml and 16 ml of non-essential AA solution and 1 ml, 4 ml and 8ml of essential AA solution per 100 ml of LDM, with or without glucose. Culture medium was then made PH neutral through the addition of NaOH. Addition of glycine or any other of the single AAs was accomplished by adding 20 g/l of each AA from powder (Sigma). PSC derived cells were differentiated in LDM until D12. AA supplementation (AA1, AA2 and AA3) was performed from day 12 until day 20 or day 30 of differentiation. Removal of glucose and/or addition of glycine (or other single AAs) at 20 g/l was done from D14 onwards. HepG2, HUH7.5 and HEK293 cells were maintained in DMEM supplemented with 10 % FBS as shown in supplementary table **ST6.** AA supplementation consisted of addition of 16 ml of non-essential amino acid mix and 8 ml of essential amino acid mix per 100 ml of medium and a further addition of 20g/l of any of the single AAs. Whenever glycine was added also DMSO was removed from the culture media. Hepatoma cell lines were plated in normal culture medium, with addition of AA3 + 20g/l AA once cells reached 60% confluency, and this for 3 or 7 additional days of culture. PHHs were maintained in a medium composition that was based on William's E basal medium as detailed in³⁹

### RNA extraction and quantitative reverse-transcription PCR (qRT-PCR).

RNA extraction was performed using the GenElute Mammalian Total RNA Miniprep Kit (Sigma-Aldrich). After isolation of genomic DNA was eliminated using the On-Column DNase I Digestion kit (Sigma-Aldrich). Last 1µg of RNA was transcribed to cDNA using the Superscript III First-Strand synthesis (Invitrogen). Gene expression analysis was performed using the Platinum SYBR green qPCR supermix-UDG kit (Invitrogen) in a ViiA 7 Real-Time PCR instrument (Thermo Fisher Scientific, Waltham, MA). The sequences of all used qRT-PCR primers are listed in supplementary table **ST4.** Of these, ribosomal protein L19 (*RPL19*) was used as a housekeeping gene for normalization.

### RNA seq Analysis

RNA sequencing fastq files were collected and reads were trimmed with Cutadapt 1.14. Adapters, low quality bases (i.e. Phred score < 20), ambiguous bases (cut-off 0.1) and polyA bases were removed from the reads. STAR 2.5.0a was used to generate the human genome index from the Grch38 assembly (release 90) available in the Ensembl database. Subsequently, STAR 2.5.0a was used to align the reads to the human genome. Read counts were summarised using the Rsubread package. Read counts were normalised for total exon length (in kb) and sequencing depth as to obtain the RPKM values. For the construction of heatmaps, we subsequently calculated the log10 values and scaled these values.

### Weighted gene correlation network analysis (WGCNA)

We queried ArrayExpress for microarrays of both stem cell-derived hepatocytes and primary hepatocytes. Only samples that were not of cancerous origin and that were labelled with biotin were used for analysis. Samples were RMA normalised per platform, followed by quantile normalisation to remove platform effects. Probe intensities were summarised by the geometric mean. Only genes that were significantly differentially expressed (Holm-Bonferroni adjusted p-value < 0.05) between hepatocyte-like cells and primary hepatocytes were kept for analysis. Furthermore, we applied a cut-off on the expression range. Transcription factors differing less than twofold over all samples were removed, as well as other genes differing less than fourfold over all samples. Using this cleaned dataset, we perform a signed weighted gene correlation network analysis (WGCNA) analysis as described by Langfelder and collegues⁵⁶. A soft-thresholding power of 19 was used as to approach scale-free topology (R2= 0.864) and highly correlated modules were merged (cut height 0.2). To identify which pathways were dysregulated in each cluster, we performed gene ontology analysis using the TopGO R package and KEGG pathway enrichment analysis using the clusterProfiler R package.

### iRegulon search

Affymetrix microarrays (HG-U133Plus2) of primary human hepatocytes were obtained from⁵⁷ (EMBL-EBI accession: E-MTAB-3994). Microarrays of both freshly isolated hepatocytes and 14-day collagen-cultured hepatocytes were used for analysis. Microarrays were analysed in R using the 'limma', 'affy', and 'oligo' packages. Benjamini-Hochberg multiple-test correction was applied (FDR < 0.05) and genes with at least 1.5-fold difference were selected. Selected genes were analysed with the iRegulon Cytoscape program ¹⁹ to identify enriched motifs in their cis-regulatory control elements. Candidate transcription factors were ranked by number of target genes in the dataset.

### Seahorse measurements

Oxygen consumption and extracellular acidification were analyzed by differentiating the cells in Seahorse XF24 tissue culture plates (Seahorse Bioscience Europe, Copenhagen, Denmark). The measurement of oxygen consumption rate (OCR) was performed at 10 min intervals (2 min mixing, 2 min recovery, 6 min measuring) for 3 h using the Seahorse XF24 analyzer. Cells were exposed to 1µM of oligomycin (Oligo) to correlate oxygen consumption with mitochondrial activity, to 1.5 µM of carbonyl cyanide-4-(trifluoromethoxy) phenylhydrazone (FCCP), an uncoupler of the electron transport chain, to measure maximal respiration, and finally to 1 µM of antimycin (Anti). Data was then normalized for protein content, quantified using the Pierce^{™} BCA Protein Assay Kit (Thermos Scientific, 23225) following manufacturer's instructions.

### GC and HPLC MS measurements

Metabolite extraction, derivatization and measurement on the GC-MS was done as described previously⁵⁸. Briefly cellular metabolism was quenched in liquid nitrogen. Metabolites were then obtained through a cold two-phase methanol-water-chloroform extraction and phase separation at 4°C, dried using a vacuum concentrator and stored at -80°C. Sample derivation was accomplished by a reaction with 7.5 µl of methoxyamine (20 mg/ml in pyridine) for 90 min at 37°C and with 15 µl of N-(tert-butyldimethylsilyl)-N-methyl-trifluoroacetamide for 60 min at 60°C. Metabolite levels and isotopomer distribution was then measured with a 7890A GC system (Agilent Technologies). Isotopomer distributions were extracted from the raw ion chromatograms using a custom Matlab M-file. Isotopomers were then calculated as a fraction of the total amount. Glucose, pyruvate and lactate concentrations were measured in the media samples high performance liquid chromatography (HPLC). Medium samples were taken at different time points and stored at -80°C. They were measured on a HPLC Bio Rad organic acid analysis column, Aminex HPX-87H, Ion exclusion column, 300mmx7.8mm with 5mM H2SO4 as the mobile phase. Peeks were integrated manually, as was a standard curve for glucose, lactate and pyruvate made in basal medium. For tissue samples, tissues were weighed (5-10 mg) and pulverized (Cryomill, Retsch) under liquid nitrogen conditions. Next, -20°C cold 65% methanol was added to the samples, followed by -20°C cold chloroform. Samples were then processed as described above. (GC-MS measurement) For tissue extracts, the total ion counts were normalized to the internal standard and tissue weight.

### Functional assessments

Urea concentrations in the media were analyzed after 24h of culture using the QuantiChromTM Urea Assay Kit (BioAssay Systems DIUR-500) according to manufacturer's instructions. CYP3A4 dependent metabolisation was determined using the fluorimetric probe 7-benzyloxy-4-trifluoromethylcoumarin (BFC) as described by⁵⁹. Metabolisation was assessed for 1h and 4h incubation times. For PHHs and datapoints which showed to high values, samples were diluted 1/10 in buffer. The albumin secretion rate was quantified using the human albumin ELISA quantitation kit (Bethyl) according to instructions provided in the kit.

### Western blot analysis

Cells were collected by trypsinisation and then lysed in RIPA lysis and extraction buffer (Thermo Scientific) supplemented with proteinase (complete, Mini Protease Inhibitor Cocktail Tablets provided in a glass vial, Roche, 11836153001) and phosphatase (PhosSTOP^{™}, Sigma, 4906845001). Protein amount was measured using a pierce BCA protein assay kit (Thermo Scientific). Alliquots of 25 µg of protein were loaded on a NuPAGE 4-12% denaturing Bis-Tris gel and transferred to a nitrocellulose membrane (Thermo Scientific). Membranes were incubated overnight at 4°C with primary antibodies (supplementary table **ST4**), and afterwards incubated with horseradish peroxidase-linked mouse secondary antibodies (Cell Signaling Technology #7076), and bound antibodies visualized using Pierce ECL reagent (ThermoFisher Scientific).

### Toxicity measurements upon drug administration

PSC, HC3X and HC6X cells were differentiated in a 384 well format. Cells were treated with 14 different concentrations of compounds in 6 repeats. As a negative control 41 wells were left untreated. After 24h of exposure media was aspirated and fresh media was added to the cells supplemented with 0.3mM of DRAQ 7 (Abcam; ab109202) and Hoechst dye (1/1000, Sigma, 33258) for 25 min at 37°. Cells were fixed with 4% paraformaldehyde. Imaging was done using an Operetta High-Content Imaging System (Perkin Elmer) and image analysis was performed using Harmony software (Perkin Elmer). Relative cell number was calculated as the percentage of living cells (draq7 positive amount subtracted from the Hoechst positive amount) relative to the average of living cells in the control wells. As a positive control, 2 donors of PHHs were seeded at 10 000 cells per well. Drugs were administrated after 8 hours of attachment. HepG2 cells were seeded at 40 000 cells per well. For control conditions HepG2 cells were exposed after 2 days of culture. For AAGLY conditions HepG2 cells were maintained in the supplemented medium for 30 days before exposure.

### Cardiomyocyte differentiation

Differentiation of cardiomyocytes was performed as described by Duelen et al⁶⁰. Amin oacids were supplemented to the basal medium as was done for hepatocytes. AAGLY-supplemented medium was added at day 8 of differentiation and kept until day 35.

### Statistics

Comparisons between two data groups (with n ≥ 3 independent experiments) were analyzed using an unpaired two-tailed Student's t test. Values of less than 0.05 were considered significant and are indicated in the graphs as *p < 0.05, **p < 0.01, or ***p < 0.001 (or as represented with # in certain figures). All data represent the mean ± SEM (n ≥ 3).

**Table 2. Media composition**

| **HepG2** | Catalog number | Supplier | Volume for 500 ml (ml) |
|---|---|---|---|
| DMEM LG | 31885 | Invitrogen | 445 |
| FBS | F7524 | Sigma | 50 |
| Pen/Strep | 15140 | Invitrogen | 5 |

| **Huh7.5** | Catalog number | Supplier | Volume for 500 ml (ml) |
|---|---|---|---|
| DMEM HG | 41965 | Invitrogen | 440 |
| FBS | F7524 | Sigma | 50 |
| Pen/Strep | 15140 | Invitrogen | 5 |
| NEAA | 11140 | Invitrogen | 5 |

| **HEK293T** | Catalog number | Supplier | Volume for 500 ml (ml) |
|---|---|---|---|
| DMEM HG | 41965 | Invitrogen | 429 |
| FBS | F7524 | Sigma | 50 |
| Pen/Strep | 15140 | Invitrogen | 5 |
| L-Glutamine | 25030 | Invitrogen | 10 |
| Sodium Pyruvate | 11360 | Invitrogen | 6 |

| **Liver Differenti ation Media (LDM)** | Catalog number | Supplier | Volume for 500 ml (ml) |
|---|---|---|---|
| DMEM LG | 31885 | Invitrogen | 285 |
| MCDB pH=7.2 | M6770 | Sigma | 200 |
| Pen/Strep | 15140 | Invitrogen | 5 |
| L-Ascorbic Acid | A8960 | Sigma | 5 |
| ITS | 4140-045 | Invitrogen | 1.25 |
| LA-BSA | L9530 | Sigma | 1.25 |
| b-Mercapto | 31350 | Invitrogen | 0.5 |
| Dexameta sone | D-2915 | Sigma | 2 |

### REFERENCES

1. Li. Chem. Biol. Interact. 150, 3-7 (2004).
2. Godoy et al. Arch. Toxicol. 90, 2513-2529 (2016).
3. Guo et al.. Drug Metab. Dispos. 39, 528-538 (2011).
4. Anthérieu et al. Toxicology in Vitro (2012). doi:10.1016/j.tiv.2012.05.008
5. Levy, G. et al. Long-term culture and expansion of primary human hepatocytes. Nat Biotech 33, 1264-1271 (2015).
6. Gao & Liu Cell Biol. Toxicol. 1-15 (2017). doi:10.1007/s10565-017-9383-z
7. Helse et al. J. Hepatol. 64, 565-573 (2016).
8. Choudhury et al. Sci. Rep. 7, 41238 (2017).
9. Vanhove et al. Stem cell reports 7, 192-206 (2016).
10. Nakamori et al. Biochem. Biophys. Res. Commun. 469, 424-429 (2016).
11. Berger, et al. Hepatology 61, 1370-1381 (2015).
12. Bell et al. Drug Metab. Dispos. (2017).
13. Freyer et al. Biores. Open Access 5, 235-248 (2016).
14. Gu et al. Cell Stem Cell 19, 476-490 (2016).
15. Carey et al. Nature 518, 413-416 (2015).
16. Yanes et al. Metabolic oxidation regulates embryonic stem cell differentiation. Nat. Chem. Biol. 6, 411-417 (2010).
17. Zhao et al.. Cell research 23, 157-161 (2013).
18. Ordovas et al. Stem cell reports 5, 918-931 (2015).
19. Janky. et al. PLoS Comput. Biol. 10, e1003731 (2014).
20. Miksys et al. Drug Metab. Dispos. 33, 1495-1502 (2005).
21. Fijnvandraat et al. Cardiovasc. Res. 58, 399-409 (2003).
22. Cao et al. PLoS One 3, e3474 (2008).
23. Lesnefsky et al. Circ. Res. 118, 1593-1611 (2016).
24. Rana et al. Toxicol. Sci. 130, 117-131 (2012).
25. Dai et al. Stem Cells Int. 2017, 5153625 (2017).
26. Tohyam et al. Cell Stem Cell 12, 127-137 (2013).
27. Kim et al. Stem Cells 33, 2699-2711 (2015).
28. Wanet et al. Int. J. Biochem. Cell Biol. 54, 174-185 (2014).
29. Grattagliano et al.Curr. Drug Targets 12, 879-893 (2011).
30. Hopkinson, et al. Oxid. Med. Cell. Longev. 2017, 5080128 (2017).
31. Guijas et al. Nat. Biotechnol. 36, 316 (2018).
32. Shirak et al. Cell Metab. 19, 780-794 (2017).
33. Kim et al. J. Toxicol. Sci. 39, 717-723 (2014).
34. Winnike et al. Metabolomics 8, 34-49 (2012).
35. Nakamura T. Biochem. Biophys. Res. Commun. 122, 884-891 (1984).
36. Li In Vitro Cell. Dev. Biol. Anim. 31, 867-870 (1995).
37. Hutson et al. Am. J. Physiol. 252, E291-8 (1987).
38. Rashid et al. J. Clin. Invest. 120, 3127-3136 (2010).
39. Shulman & Nahmias in (eds. Randell, S. H. & Fulcher, M. L.) 287-302 (Humana Press, 2013). doi:10.1007/978-1-62703-125-7_17
40. Hannan et al. Nat. Protoc. 8, 430-437 (2013).
41. Avior et al. Hepatology 62, 265-278 (2015).
42. Chen et al. Stem cell reports 5, 22-30 (2015).
43. Peters et al. Development 143, 1475-1481 (2016).
44. Novik et al. Toxicol. Appl. Pharmacol. 336, 20-30 (2017).
45. Mitaka Cell. Physiol. 147, 495-504 (1991).
46. Taya et al. Science. 2016 354, 1152-1155.
47. Barle et al. Clin. Physiol. 16, 217-227 (1996).
48. Mallette et al. J. Biol. Chem. 244, 5713-5723 (1969).
49. Dardevet et al. Am. J. Clin. Nutr. 88, 986-996 (2008).
50. Kamalian et al. Toxicol. Vitr. 29, 732-740 (2015).
51. Gerets et al. Cell Biol. Toxicol. 28, 69-87 (2012).
52. Heslop et al. Arch. Toxicol. 91, 439-452 (2017).
53. Pradip et al. Stem Cells Int. 2016, 2475631.
54. Bell et al. Injury. Drug Metab. Dispos. 45, 419-429 (2017).
55. Ordovas et al. J. Vis. Exp. (2016). doi:10.3791/54718
56. Langfelder et al. BMC Bioinformatics 9, 559 (2008).
57. Camero et al. Stem Cell Reports 5, 1250-1262 (2017).
58. Elia et al. Nat. Commun. 8, 15267 (2017).
59. Donato et al. Drug Metab. Dispos. 32, 699-706 (2004).
60. Duelen, R. et al. Stem Cells Int. 2017, 4651238 (2017). Baxter, et al. (2015) J Hepatol 62, 581-589.

Godoy et al. (2015). J Hepatol 63, 934-942.
Godoy et al (2016) Arch Toxicol. 2513-2529.
Raju et al. (2014) Stem Cells Dev 23, 124-131.
Takebe, T. et al. (2013). Nature 499, 481-484.

## Claims

1. A method for inducing or maintaining a mature hepatocyte phenotype in a cell, the method comprising the step of cultivating cells in a cell culture medium comprising at least 10 mg amino acids/ml medium,
wherein at least 50 % (w/w) of the concentration of said amino acids in said medium is provided by one up to five amino acids and, with the proviso that said one up to five amino acids is not one of Thr, Tyr, Cys, Met, Arg and His.

2. The method according to claim 1, wherein said one up to five amino acids are selected from the group consisting of Gly, Ala, Ser Val, Ile, Leu, Pro, Asp and taurine.

3. The method according to claim 1 or 2, wherein at least 50 % (w/w) of the concentration amino acids in said medium is Glycine.

4. The method according to any one of claims 1 to 3, comprising the step of determining a marker of mature phenotype selected from the group consisting of albumin secretion, CYP450 activity, glycogen storage, susceptibility to infection with hepatotropic viruses, drug metabolising capacities and expression of NTCP, HNF4A and AAT.

5. The method according to any one of claims 1 to 4, wherein the cultivation in said medium is performed for at least 5 days.

6. The method according to any one of claims 1 to 5, wherein said cultivated cells are selected from the group consisting of partially or fully differentiated hepatocyte stem cells, hepatocyte progenitor cells or precursor cells, freshly isolated or cultivated hepatocyte cells, primary liver tumour cells or immortalised liver tumour cells.

7. The method according to 6, wherein the stem cells are PSC.

8. The method according to claims 6 or 7, further comprising the step of transfecting said stem cells with one or more transcription factors selected from the group of *HNF1A, FOXA3* and *PROX1.*

9. A cell culture medium comprising **characterised in** the presence of at least 10 mg amino acids/ml medium wherein at least 70 %, (w/w) of the concentration of said amino acids in said medium is provided by one up to five amino acids and, with the proviso that said one up to five amino acids is not one of Thr, Tyr, Cys, Met, Arg or His.

10. The medium according to claim 9, wherein the medium comprises at least 15 mg amino acids/ml medium.

11. The medium according to claim 9 or 10, wherein said one up to five amino acids are selected from the group consisting of Gly, Ala, Ser Val, Ile, Leu, Pro, Asp and taurine.

12. The medium according to any one of claims 9 to 11, wherein at least 50 % (w/w) of the concentration amino acids in said medium is Glycine.

13. In vitro use of a cell culture medium **characterised in** the presence of at least 10 mg amino acids/ml medium wherein at least 50 %, (w/w) of the concentration of said amino acids in said medium is provided by one up to five amino acids and, with the proviso that said one up to five amino acids is not one of Thr, Tyr, Cys, Met, Arg or His,
for maintaining or inducing mature hepatocyte phenotype in cells, for inducing or maintaining mitochondrial activity in a cell, of for inducing or maintaining mitochondrial activity in a cardiomyocyte or kidney epithelial cell

14. A method for the identification of a medium suitable for inducing or maintaining a mature hepatocyte phenotype in a cell comprising the steps of:
- providing a test medium with one or more amino acids at determined concentrations wherein the total concentration of amino acids in said medium is at least 10 mg/ml,
- cultivating the cells in the presence of said test medium,
- determining one or more markers for a mature phenotype of hepatocytes,
- selecting a medium, wherein the presence of such one or more parameters is determined, as suitable inducing or maintaining a mature hepatocyte phenotype in a cell.

15. The method according to claim 14 wherein the marker a mature phenotype of hepatocytes is CYP450 activity.

## Patentansprüche

1. Verfahren zum Induzieren oder Aufrechterhalten eines reifen Hepatozytenphänotyps in einer Zelle, das Verfahren umfassend den Schritt des Kultivierens von Zellen in einem Zellkulturmedium, umfassend mindestens 10 mg Aminosäuren/ml Medium, wobei mindestens 50 Gew.-% der Konzentration der Aminosäuren in dem Medium durch eine bis zu fünf Aminosäuren bereitgestellt werden, mit der Maßgabe, dass es sich bei der einen bis zu fünf Aminosäuren nicht um eine von Thr, Tyr, Cys, Met, Arg und His handelt.

2. Verfahren nach Anspruch 1, wobei die eine bis zu fünf Aminosäuren aus der Gruppe ausgewählt sind, bestehend aus Gly, Ala, Ser, Val, Ile, Leu, Pro, Asp und Taurin.

3. Verfahren nach Anspruch 1 oder 2, wobei mindestens 50 Gew.-% der Aminosäurekonzentration in dem Medium Glycin sind.

4. Verfahren nach einem der Ansprüche 1 bis 3, umfassend den Schritt des Bestimmens eines Markers des reifen Phänotyps, der aus der Gruppe ausgewählt ist, bestehend aus Albuminsekretion, CYP450-Aktivität,
Glykogenspeicherung, Anfälligkeit für eine Infektion mit hepatotropen Viren, Arzneimittelmetabolisierungskapazitäten und Expression von NTCP, HNF4A und AAT.

5. Verfahren nach einem der Ansprüche 1 bis 4, wobei die Kultivierung in dem Medium für mindestens 5 Tage durchgeführt wird.

6. Verfahren nach einem der Ansprüche 1 bis 5, wobei die kultivierten Zellen aus der Gruppe ausgewählt sind, bestehend aus teilweise oder vollständig differenzierten Hepatozytenstammzellen, Hepatozytenprogenitorzellen oder - vorläuferzellen, frisch isolierten oder kultivierten Hepatozytenzellen, primären Lebertumorzellen oder immortalisierten Lebertumorzellen.

7. Verfahren nach 6, wobei die Stammzellen PSC sind.

8. Verfahren nach den Ansprüchen 6 oder 7, ferner umfassend den Schritt des Transfizierens der Stammzellen mit einem oder mehreren Transkriptionsfaktoren, die aus der Gruppe von *HNF1A, FOXA3* und *PROX1* ausgewählt sind.

9. Zellkulturmedium, umfassend **gekennzeichnet durch** die Anwesenheit von mindestens 10 mg Aminosäuren/ml Medium, wobei mindestens 70 Gew.-% der Konzentration der Aminosäuren in dem Medium durch eine bis zu fünf Aminosäuren bereitgestellt werden, mit der Maßgabe, dass es sich bei der einen bis zu fünf Aminosäuren nicht um eine von Thr, Tyr, Cys, Met, Arg oder His handelt.

10. Medium nach Anspruch 9, wobei das Medium mindestens 15 mg Aminosäuren/ml Medium umfasst.

11. Medium nach Anspruch 9 oder 10, wobei die eine bis zu fünf Aminosäuren aus der Gruppe ausgewählt sind, bestehend aus Gly, Ala, Ser, Val, Ile, Leu, Pro, Asp und Taurin.

12. Medium nach einem der Ansprüche 9 bis 11, wobei mindestens 50 Gew.-% der Aminosäurekonzentration in dem Medium Glycin sind.

13. In-vitro-Verwendung eines Zellkulturmediums, **gekennzeichnet durch** die Anwesenheit von mindestens 10 mg Aminosäuren/ml Medium, wobei mindestens 50 Gew.-% der Konzentration der Aminosäuren in dem Medium durch eine bis zu fünf Aminosäuren bereitgestellt werden und mit der Maßgabe, dass es sich bei der einen bis zu fünf Aminosäuren nicht um eine von Thr, Tyr, Cys, Met, Arg oder His handelt,
zum Aufrechterhalten oder Induzieren eines reifen Hepatozytenphänotyps in Zellen, zum Induzieren oder Aufrechterhalten mitochondrialer Aktivität in einer Zelle, oder zum Induzieren oder Aufrechterhalten mitochondrialer Aktivität in einer Kardiomyozyten- oder Nierenepithelzelle.

14. Verfahren zur Identifizierung eines Mediums, das zum Induzieren oder Aufrechterhalten eines reifen Hepatozytenphänotyps in einer Zelle geeignet ist, umfassend die Schritte:
- Bereitstellen eines Testmediums mit einer oder mehreren Aminosäuren in bestimmten Konzentrationen, wobei die Gesamtkonzentration an Aminosäuren in dem Medium mindestens 10 mg/ml beträgt,
- Kultivieren der Zellen in Gegenwart des Testmediums,
- Bestimmen eines oder mehrerer Marker für einen reifen Phänotyp von Hepatozyten,
- Auswählen eines Mediums, wobei das Vorhandensein eines oder mehrerer solcher Parameter bestimmt wird, die zum Induzieren oder Aufrechterhalten eines reifen Hepatozytenphänotyps in einer Zelle geeignet sind.

15. Verfahren nach Anspruch 14, wobei der Marker eines reifen Phänotyps von Hepatozyten CYP450-Aktivität ist.

## Revendications

1. Procédé permettant d'induire ou de maintenir un phénotype d'hépatocytes mature dans une cellule, le procédé comprenant l'étape de culture de cellules dans un milieu de culture cellulaire comprenant au moins 10 mg d'acides aminés/ml de milieu, dans lequel au moins 50 % (en poids) de la concentration desdits acides aminés dans ledit milieu est fournie par un à cinq acides aminés et, à condition que celui précité des un à cinq acides aminés ne soit pas l'un parmi Thr, Tyr, Cys, Met, Arg et His.

2. Procédé selon la revendication 1, dans lequel ceux précités des un à cinq acides aminés sont choisis dans le groupe constitué par Gly, Ala, Ser Val, Ile, Leu, Pro, Asp et taurine.

3. Procédé selon la revendication 1 ou 2, dans lequel au moins 50 % (en poids) de la concentration d'acides aminés dans ledit milieu est Glycine.

4. Procédé selon l'une quelconque des revendications 1 à 3, comprenant l'étape de détermination d'un marqueur de phénotype mature choisi dans le groupe constitué par sécrétion d'albumine, activité de CYP450, stockage de glycogène, susceptibilité à une infection par des virus hépatotropes, capacités de métabolisation de médicaments et expression de NTCP, HNF4A et AAT.

5. Procédé selon l'une quelconque des revendications 1 à 4, dans lequel la culture dans ledit milieu est mise en oeuvre pendant au moins 5 jours.

6. Procédé selon l'une quelconque des revendications 1 à 5, dans lequel lesdites cellules cultivées sont choisies dans le groupe constitué par cellules souches d'hépatocytes partiellement ou totalement différenciées, cellules progénitrices ou cellules de précurseur d'hépatocytes, cellules d'hépatocytes fraîchement isolées ou cultivées, cellules tumorales hépatiques primaires ou cellules tumorales hépatiques immortalisées.

7. Procédé selon 6, dans lequel les cellules souches sont PSC.

8. Procédé selon les revendications 6 ou 7, comprenant en outre l'étape consistant à transfecter lesdites cellules souches avec un ou plusieurs facteurs de transcription choisis dans le groupe de *HNF1A, FOXA3* et *PROX1.*

9. Milieu de culture cellulaire comprenant **caractérisé par** la présence d'au moins 10 mg d'acides aminés/ml de milieu dans lequel au moins 70 %, (en poids) de la concentration desdits acides aminés dans ledit milieu est fournie par un à cinq acides aminés et, à condition que celui précité des un à cinq acides aminés ne soit pas l'un parmi Thr, Tyr, Cys, Met, Arg ou His.

10. Milieu selon la revendication 9, dans lequel le milieu comprend au moins 15 mg d'acides aminés/ml de milieu.

11. Milieu selon la revendication 9 ou 10, dans lequel ceux précités des un à cinq acides aminés sont choisis dans le groupe constitué par Gly, Ala, Ser Val, Ile, Leu, Pro, Asp et taurine.

12. Milieu selon l'une quelconque des revendications 9 à 11, dans lequel au moins 50 % (en poids) de la concentration d'acides aminés dans ledit milieu est Glycine.

13. Utilisation in vitro d'un milieu de culture cellulaire **caractérisé par** la présence d'au moins 10 mg d'acides aminés/ml de milieu dans laquelle au moins 50 %, (en poids) de la concentration desdits acides aminés dans ledit milieu est fournie par un à cinq acides aminés et, à condition que celui précité des un à cinq acides aminés ne soit pas l'un parmi Thr, Tyr, Cys, Met, Arg ou His, permettant de maintenir ou d'induire un phénotype d'hépatocytes mature dans cellules, permettant d'induire ou de maintenir une activité mitochondriale dans une cellule, ou permettant d'induire ou de maintenir une activité mitochondriale dans un cardiomyocyte ou une cellule épithéliale rénale

14. Procédé destiné à l'identification d'un milieu approprié permettant d'induire ou de maintenir un phénotype d'hépatocytes mature dans une cellule comprenant les étapes consistant à :
- fournir à un milieu de test un ou plusieurs acides aminés à des concentrations déterminées dans lequel la concentration totale d'acides aminés dans ledit milieu vaut au moins 10 mg/ml,
- cultiver les cellules en présence dudit milieu de test,
- déterminer un ou plusieurs marqueurs pour un phénotype mature d'hépatocytes,
- sélectionner un milieu, dans lequel la présence d'un tel ou de tels paramètres est déterminée, comme approprié, induisant ou maintenant un phénotype d'hépatocytes mature dans une cellule.

15. Procédé selon la revendication 14 dans lequel le marqueur d'un phénotype mature d'hépatocytes est une activité de CYP450.
